# EUROPEAN PATENT SPECIFICATION

(11) **EP 3 505 201 B1**
(45) Date of publication and mention of the grant of the patent: **03.08.2022**
(21) Application number: 17843557.4
(22) Date of filing: 22.08.2017
(51) Int. Cl.: A61M 5/00, A61M 5/28, A61M 5/178

(54) **SYRINGE PACKAGE**
SPRITZENVERPACKUNG
EMBALLAGE DE SERINGUE.

(30) Priority: 23.08.2016 JP 2016162844; 23.08.2016 JP 2016162888; 23.08.2016 JP 2016162898
(43) Date of publication of application: 03.07.2019
(73) Proprietor: Terumo Kabushiki Kaisha, Tokyo 151-0072 (JP)
(72) Inventor: OGAWA, Junichi, Yamanashi 409-3853 (JP); KOTANI, Yoshikazu, Yamanashi 409-3853 (JP); KOIWAI, Kazunori, Kanagawa 250-0853 (JP); URA, Takehiro, Kanagawa 259-0151 (JP)
(74) Representative: Casalonga
(86) International application number: PCT/JP2017/029864
(87) International publication number: WO 2018/038077

(56) References cited:
- EP-A1- 2 682 145
- WO-A1-2012/117837
- WO-A1-2013/176703
- WO-A1-2014/045336
- JP-A- 2006 271 938
- JP-A- 2011 139 755
- JP-A- 2013 013 581
- JP-A- 2013 188 431
- JP-A- 2014 162 532
- US-A1- 2006 275 336

## Description

### Technical Field

The present invention relates to a syringe package that includes a prefilled syringe system filled with a drug in advance.

### Background Art

For example, WO 2012/117837 discloses a prefilled syringe system that includes a gasket and a plunger being spaced from each other when not in use. The prefilled syringe system includes a space maintaining unit for maintaining the space between the gasket and the plunger.

WO 2013/176703 discloses a syringe package for a prefilled syringe.

### Summary of Invention

While being transported, a prefilled syringe system is housed in a package body. At this time, it is necessary to prevent a plunger from contacting a gasket (to prevent the gasket from being pressed by the plunger) due to vibration during transportation. The prefilled syringe system disclosed in WO 2012/117837 is provided with the space maintaining unit so that the plunger is prevented from contacting the gasket due to vibration during transportation. This arrangement may be a cause of complexity of the prefilled syringe system.

The present invention has been made in light of such a problem, and an object of the present invention is to provide a syringe package which enables a prefilled syringe system with a simple arrangement and which prevents a gasket from being pressed by a plunger during transportation.

In order to achieve the object, a syringe package according to an embodiment of the present invention is provided with a prefilled syringe system that includes a prefilled syringe including a syringe body which is filled with a drug in advance and in which a gasket and a plunger are not connected to each other when the syringe package is not in use, wherein the syringe package includes: a package body that houses the prefilled syringe; and a support disposed inside the package body and configured to support the prefilled syringe, wherein the support includes a displacement regulation unit configured to regulate relative displacement of the syringe body and the plunger in a direction in which the gasket and the plunger approach each other.

According to such an arrangement, the package body includes the displacement regulation unit. Accordingly, it is possible to simplify the arrangement of the prefilled syringe system and to prevent the gasket from being pressed by the plunger during transportation of the syringe package.

In the syringe package, the gasket and the plunger are spaced from each other.

Such an arrangement further prevents the gasket from being pressed by the plunger during transportation of the syringe package.

In the syringe package, the plunger has a base end portion provided with a plunger flange, and the displacement regulation unit is disposed between the syringe body and the plunger flange.

Such an arrangement allows the displacement regulation unit to effectively prevent displacement of the syringe body toward a base end (the plunger flange) with respect to the plunger and displacement of the plunger toward a leading end (the gasket) with respect to the syringe body.

In the syringe package, the displacement regulation unit may be provided with a groove into which at least a part of the plunger is inserted.

Such an arrangement prevents separation of the displacement regulation unit from between the syringe body and the plunger flange due to vibration during transportation.

In the syringe package, the support may include a base that holds a position of the displacement regulation unit inside the package body.

Such an arrangement prevents displacement of the displacement regulation unit with respect to the package body.

In the syringe package, the support may include a positional shift regulation unit that contacts the syringe body and regulates displacement of the syringe body in a direction intersecting with an axis of the syringe body.

Such an arrangement further prevents separation of the displacement regulation unit from between the syringe body and the plunger flange due to vibration during transportation.

In the syringe package, the positional shift regulation unit may lock the syringe body.

With a simple arrangement, such an arrangement more effectively prevents separation of the displacement regulation unit from between the syringe body and the plunger flange due to vibration during transportation.

In the syringe package, the positional shift regulation unit may be provided on the base.

Such an arrangement prevents displacement of the positional shift regulation unit with respect to the package body.

In the syringe package, the support may include a leading end regulation unit that contacts the syringe body and regulates displacement of the syringe body toward a leading end of the syringe body.

Such an arrangement enables the leading end regulation unit to further prevent a positional shift of the syringe body.

In the syringe package, the leading end regulation unit may be provided on the base.

Such an arrangement prevents displacement of the leading end regulation unit with respect to the package body.

In the syringe package, the prefilled syringe system may include: a needle package including a needle unit that enables puncture of a living body and a needle unit case that houses the needle unit; and the prefilled syringe including a connection to which the needle unit is connected, the syringe body, and the plunger, wherein the support includes a needle unit case support that supports the needle unit case.

Such an arrangement makes it possible to support the needle package and the prefilled syringe inside the package body.

In the syringe package, the needle unit and the prefilled syringe may be separated from each other when not in use.

Such an arrangement makes it possible to individually support the needle package and the prefilled syringe inside the package body.

In the syringe package, the needle unit case support may be provided on the base.

Such an arrangement prevents displacement of the needle unit case support with respect to the package body.

According to an embodiment of the present invention, the package body includes the displacement regulation unit. Accordingly, it is possible to simplify the arrangement of the prefilled syringe system and to prevent the gasket from being pressed by the plunger.

### Brief Description of Drawings

Fig. 1 is an exploded perspective view of a syringe package according to a first embodiment of the present invention.
Fig. 2 is a longitudinal sectional view of a prefilled syringe included in the syringe package.
Fig. 3 is a longitudinal sectional view of a needle package included in the syringe package.
Fig. 4 is a plan view of a support included in the syringe package.
Fig. 5 is a longitudinal sectional view of the syringe package.
Fig. 6 is a longitudinal sectional view taken along line VI-VI of Fig. 5.
Fig. 7A is a transverse sectional view taken along line VIIA-VIIA of Fig. 5, and Fig. 7B is a transverse sectional view taken along line VIIB-VIIB of Fig. 5.
Fig. 8 is an exploded perspective view of a syringe package provided with a support according to a modification.
Fig. 9 is a perspective view of the support and a prefilled syringe system illustrated in Fig. 8.
Fig. 10 is an exploded perspective view of a syringe package according to a second embodiment of the present invention.
Fig. 11 is an exploded perspective view of a support and two prefilled syringe systems illustrated in Fig. 10.
Fig. 12 is a longitudinal sectional view of the syringe package illustrated in Fig. 10.
Fig. 13 is a longitudinal sectional view taken along line XIII-XIII of Fig. 12.
Fig. 14 is an exploded perspective view of a syringe package according to a third embodiment of the present invention.
Fig. 15 is a longitudinal sectional view of the syringe package illustrated in Fig. 14.
Fig. 16 is a longitudinal sectional view taken along line XVI-XVI of Fig. 15.
Fig. 17 is a perspective view of a syringe package according to a fourth embodiment of the present invention.
Fig. 18 is an exploded perspective view of the syringe package illustrated in Fig. 17.
Fig. 19 is a cross-sectional view of the syringe package taken along the line XIX-XIX in Fig. 17.
Fig. 20 is a perspective view of a syringe package according to a fifth embodiment of the present invention.
Fig. 21 is an exploded perspective view of the syringe package illustrated in Fig. 20.
Fig. 22 is a cross-sectional view of the syringe package taken along line XXII-XXII in Fig. 20.
Fig. 23 is a perspective view of a syringe package according to a sixth embodiment of the present invention.
Fig. 24 is an exploded perspective view of the syringe package illustrated in Fig. 23.
Fig. 25 is a cross-sectional view of the syringe package taken along line XXV-XXV in Fig. 23.
Fig. 26 is an exploded perspective view of a syringe package according to a reference example of the present invention.
Fig. 27 is a plan view of a support illustrated in Fig. 26.
Fig. 28 is a longitudinal sectional view of the syringe package of Fig. 26.
Fig. 29 is a cross-sectional view of the syringe package taken along line XXIX-XXIX in Fig. 28.

### Description of Embodiments

Preferred embodiments of a syringe package according to the present invention will now be described with reference to the accompanying drawings.

### (First Embodiment)

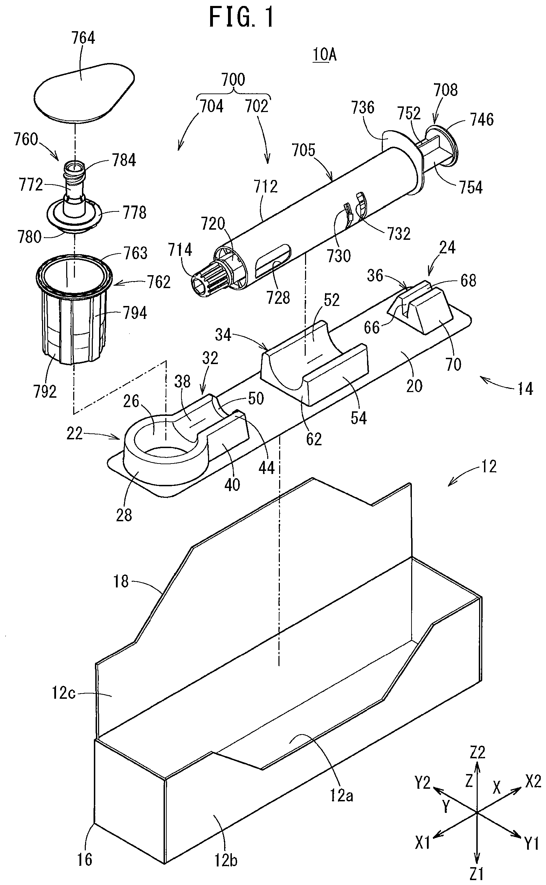

As illustrated in Fig. 1, a syringe package 10A according to a first embodiment of the present invention includes a prefilled syringe system 700, a package body 12, and a support 14. The prefilled syringe system 700 includes a tubular syringe body 705 (inner cylinder 710) filled with a predetermined drug in advance. The package body 12 houses the syringe system 700. The support 14 is disposed inside the package body 12 and supports the prefilled syringe system 700.

First, the prefilled syringe system 700 will be described. Figs. 1 to 3 illustrate the prefilled syringe system 700 as a device for subcutaneous or intradermal administration of a vaccine (drug) for influenza or the like. The prefilled syringe system 700 includes a prefilled syringe 702 and a needle package 704 provided with a needle unit 760. Specifically, when not in use, the prefilled syringe system 700 is kept while the prefilled syringe 702 and the needle unit 760 are separated from each other, and when in use, the needle unit 760 of the needle package 704 is attached to the prefilled syringe 702.

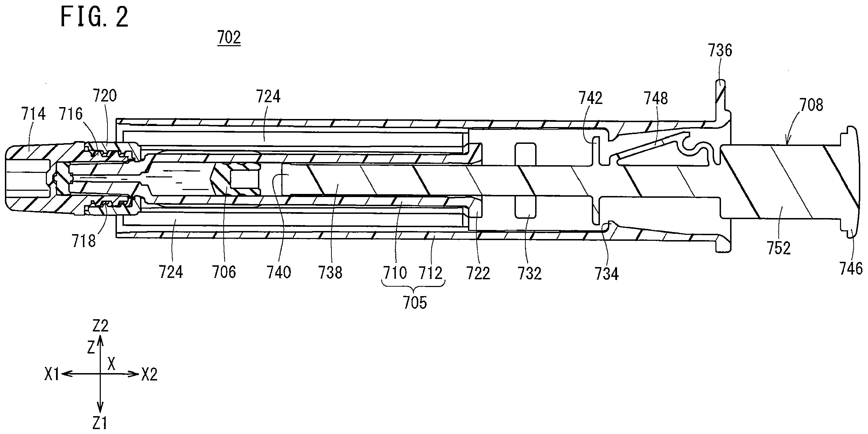

As illustrated in Figs. 1 and 2, the prefilled syringe 702 includes the tubular syringe body 705, a gasket 706 provided inside the syringe body 705, and a plunger 708 that presses the gasket 706 toward a leading end of the syringe body 705. The syringe body 705 includes an inner cylinder 710, and an outer cylinder 712. The inner cylinder 710 is included in a small diameter barrel filled with the drug. The outer cylinder 712 is included in a large diameter cowling cylinder provided on an outer periphery of the inner cylinder 710.

The inner cylinder 710 is formed in an integrated manner, including, for example, a resin material or glass. The inner cylinder 710 is transparent so as to make a liquid drug inside the inner cylinder 710 visible. A leading end portion of the inner cylinder 710 has a diameter smaller than a base end of the inner cylinder 710. To the leading end portion of the inner cylinder 710, a connection 720 is fixed. The connection 720 includes a threaded portion 718 into which a threaded portion 716 of a cap 714 is screwed. The cap 714 is configured to seal an opening portion at a leading end of inner cylinder 710. The base end of the inner cylinder 710 is provided with an inner cylinder flange 722.

The outer cylinder 712 is formed in an integrated manner, including a resin material. It is preferable that the outer cylinder 712 be transparent in order to easily make the liquid drug filled inside the inner cylinder 710 visible. The outer cylinder 712 is provided coaxially with the inner cylinder 710. A leading end of the outer cylinder 712 is placed somewhat closer to a base end of the syringe body 705 than the leading end of the inner cylinder 710. In other words, a part of the connection 720 of the inner cylinder 710 is exposed to the leading end of the outer cylinder 712. Furthermore, the outer cylinder 712 is extended toward the base end of the syringe body 705 from the base end of the inner cylinder 710.

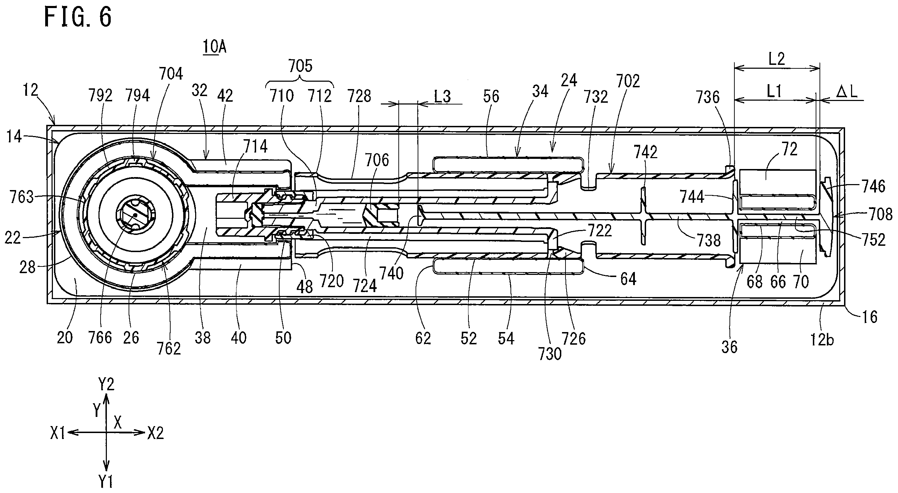

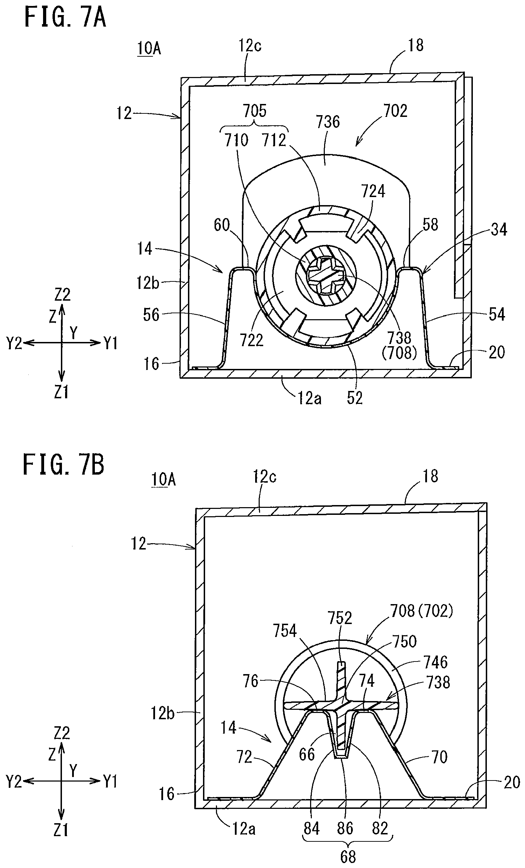

An inner periphery of the outer cylinder 712 is provided with a plurality of (four) leading end protrusions 724 (see Fig. 7A) and a plurality of (two) base end protrusions 726 (see Fig. 6) for supporting the inner cylinder 710. The leading end protrusions 724 are in contact with a leading end surface of the inner cylinder flange 722. The base end protrusions 726 are in contact with a base end surface of the inner cylinder flange 722. In other words, the leading end protrusions 724 and the base end protrusions 726 clamp the inner cylinder flange 722 in the direction of axis so that the inner cylinder 710 is fixed to the outer cylinder 712.

As illustrated in Figs. 1 and 6, the outer cylinder 712 has an outside diameter which enables a user to hold the outer cylinder 712 easily in his/her hand. On an outer periphery of the outer cylinder 712, first opening portions 728, second opening portions 730, and third opening portions 732 are formed in pairs, and the opening portions of each pair face each other. The first opening portions 728, the second opening portions 730, and the third opening portions 732 are aligned along the axis of the outer cylinder 712.

Each first opening portion 728 is a hole that enables visual check of the drug inside the inner cylinder 710 and is provided at the leading end of the outer cylinder 712. In the illustrated example, each first opening portion 728 is formed as an elongated hole extended in the direction of axis of the outer cylinder 712. It should be noted that each first opening portion 728 may be formed in any shape.

Each second opening portion 730 is a hole that enables formation of the leading end protrusions 724 and the base end protrusions 726 and is placed closer to a base end of the outer cylinder 712 than each first opening portion 728. The two second opening portions 730 face each other, sandwiching the inner cylinder flange 722. Each second opening portion 730 is extended along a circumferential direction of the outer cylinder 712. It should be noted that each second opening portion 730 may be formed in any shape.

Each third opening portion 732 is a hole that enables assembly of the plunger 708 and is placed closer to the base end of the outer cylinder 712 than each second opening portion 730. Each third opening portion 732 is extended along the circumferential direction of the outer cylinder 712. It should be noted that each third opening portion 732 may be formed in any shape.

As illustrated in Fig. 2, on the inner periphery of the outer cylinder 712, a part closer to the base end than the third opening portions 732 is provided with a projecting portion 734 that projects radially inward. The base end of the outer cylinder 712 is provided with an outer cylinder flange 736 extended radially outward from the outer cylinder 712.

The gasket 706 is provided liquid-tightly and movably along the axis of the inner cylinder 710. In other words, the gasket 706 is slidable relative to an inner periphery of the inner cylinder 710. Examples of a material of the gasket 706 include various rubber materials such as natural rubber, butyl rubber, isoprene rubber, butadiene rubber, styrene-butadiene rubber, and silicone rubber; various thermoplastic elastomers of polyurethane type, polyester type, polyamide type, olefin type, and styrene type; or elastic materials such as mixtures of the above examples.

The plunger 708 is formed in an integrated manner, including a resin material. A leading end of the plunger 708 is inserted into the inner cylinder 710, and a base end portion of the plunger 708 is exposed to the base end from the outer cylinder 712. The plunger 708 includes a shaft 738 extended along the axis of the inner cylinder 710.

A leading end of the shaft 738 is provided with a pressing portion 740 for pressing a base end surface of the gasket 706 toward the leading end of the inner cylinder 710. When not in use, the pressing portion 740 is spaced from (is not connected to) the gasket 706.

As illustrated in Figs. 2 and 6, a first intermediate flange 742, a second intermediate flange 744, and a plunger flange 746 are provided on a base end of the shaft 738. The first intermediate flange 742 and the second intermediate flange 744 are formed to have a larger diameter than the inner cylinder 710 and a smaller diameter than the outer cylinder 712. When not in use, the first intermediate flange 742 contacts the projecting portion 734 of the outer cylinder 712 in order to prevent the plunger 708 from slipping out of the inner cylinder 710. The second intermediate flange 744 is provided with a lock 748. At the completion of administrating the drug, the lock 748 is locked with the projecting portion 734 and prevents displacement of the plunger 708 along the axis.

The plunger flange 746 is exposed to the base end of the outer cylinder 712. The plunger flange 746 is a disk-shaped member having a size that enables easy manual handling, being extended radially outward from the base end of the shaft 738. The plunger flange 746 has an outside diameter larger than an inside diameter of the outer cylinder 712.

As illustrated in Figs. 7A and 7B, a transverse section of the shaft 738 is formed in a cross shape. In Fig. 7B, a section of the shaft 738 between the second intermediate flange 744 (see Fig. 6) and the plunger flange 746 includes a central portion 750, two plate-shaped first protruding portions 752, and two plate-shaped second protruding portions 754. The central portion 750 is placed on the axis of the shaft 738. The two first protruding portions 752 are extended in opposite directions from the central portion 750. The two second protruding portions 754 are extended in opposite directions from the central portion 750 in a direction perpendicular to the extension direction of the first protruding portions 752. The central portion 750, each of the first protruding portions 752, and each of the second protruding portions 754 are extended from the second intermediate flange 744 to the plunger flange 746. A length of each first protruding portion 752 extended from the central portion 750 is shorter than a length of each second protruding portion 754 extended from the central portion 750.

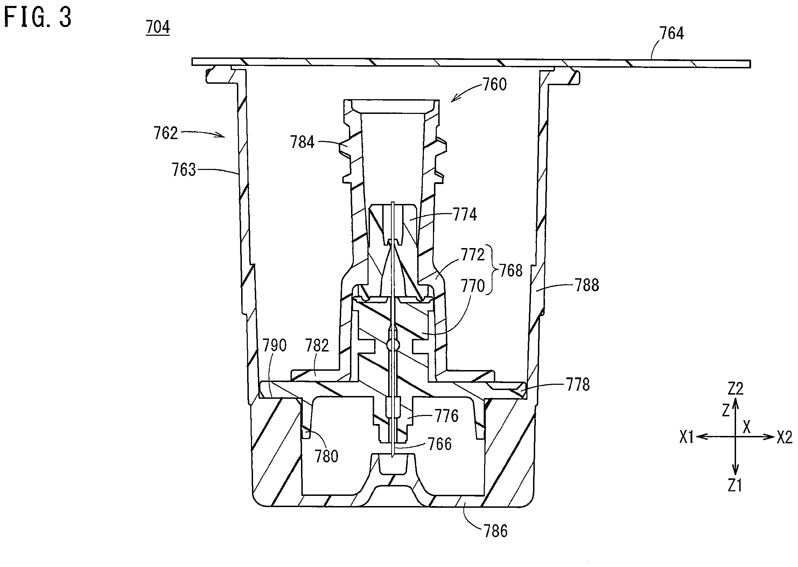

As illustrated in Figs. 1 and 3, the needle package 704 includes the needle unit 760 that is attachable to the connection 720 of the prefilled syringe 702 and a needle unit case 762 that houses the needle unit 760. The needle unit case 762 includes a case body 763 that houses the needle unit 760 and a sealing film 764 that seals the case body 763. The needle unit 760 includes a tubular needle body 766 having a leading end provided with a sharp needle point and a needle hub 768 to which the needle body 766 is fixed. Hereinafter, in the needle unit 760 and components thereof, a direction in which the needle point of the needle body 766 is placed may be referred to as "leading end", and the opposite side may be referred to as "base end".

The needle body 766 is a hollow tubular member that includes, for example, a metal material such as stainless steel, aluminum or an aluminum alloy, titanium or a titanium alloy; or a hard resin material such as polyphenylene sulfide.

The needle hub 768 includes a first hub 770, a second hub 772, and an elastic member 774. The first hub 770 holds the needle body 766, and the second hub 772 is a hollow member provided on the first hub 770 and is detachable from the connection 720 of the prefilled syringe 702. The elastic member 774 is disposed inside the second hub . The first hub 770 and the second hub 772 each include synthetic resin such as polycarbonate, polypropylene, and polyethylene.

The first hub 770 includes a needle holding unit 776, a flange 778, and an annular portion 780. The needle holding unit 776 is provided with an insertion hole through which the needle body 766 is inserted. The flange 778 has a circular ring shape, projecting radially outward from a leading end of the needle holding unit 776. The annular portion 780 is extended from the flange 778 toward the leading end.

A leading end portion of the needle holding unit 776 is placed inside the annular portion 780. It is preferable that a projecting length of the needle body 766 from the leading end of the needle holding unit 776 be set to, for example, 0.9 mm to 1.4 mm in order to reliably administer the liquid drug intradermally (dermally). The flange 778 is formed in a plate shape, having the maximum diameter of the needle hub 768. The flange 778 is placed close to the leading end of the needle body 766.

The annular portion 780 is formed in a circular ring shape so as to cover the leading end portion of the needle holding unit 776 in the circumferential direction. In the direction of axis of the needle body 766, the annular portion 780 has a projecting end face placed substantially equal to a leading end surface of the needle holding unit 776. The annular portion 780 has an outside diameter smaller than an outside diameter of the flange 778.

The second hub 772 is a tubular member extended in the direction of axis of the needle body 766, having a leading end portion provided with a flange 782 having a circular ring shape. While a base end of the needle holding unit 776 is inserted into a leading end of an inner hole of the second hub 772 and the flange 782 is in contact with the flange 778, the second hub 772 is fixed to the first hub 770. Into a base end of the inner hole of the second hub 772, the leading end portion of the inner cylinder 710 of the syringe body 705 is inserted. An outer periphery of a base end of the second hub 772 is provided with a threaded portion 784 which is screwed into the threaded portion 718 of the connection 720.

The elastic member 774 is placed on the base end of the needle holding unit 776 in the inner hole of the second hub 772. The elastic member 774 is provided with an insertion hole through which a base end portion of the needle body 766 is inserted. While the second hub 772 is attached to the connection 720 of the prefilled syringe 702, the elastic member 774 functions as a sealing member that contacts an outer periphery of the needle body 766 and an inner periphery of the second hub in a liquid-tight manner.

The case body 763 has a substantially U-shaped transverse section. Specifically, the case body 763 has a circular bottom surface 786 and a peripheral wall 788 provided on an outer edge of the bottom surface 786. The peripheral wall 788 is tapered, gradually decreasing in diameter toward the bottom surface 786. An inner surface of the peripheral wall 788 is provided with an engagement projection 790 with which the flange 778 is engaged. An outer surface of the peripheral wall 788 is provided with a plurality of (six in Fig. 6) recesses 792 and a plurality of (six in Fig. 6) projections 794 extended along the axis of the needle body 766. The recesses 792 and the projections 794 are provided alternately in the circumferential direction. The sealing film 764 is provided to close an opening portion of the needle unit case 762.

When using the prefilled syringe system 700 with such an arrangement, first, a user removes the cap 714 of the prefilled syringe 702 and peels off the sealing film 764 of the needle package 704. While placing the needle unit 760 inside the needle unit case 762, the user screws the threaded portion 718 of the connection 720 of the prefilled syringe 702 into the threaded portion 784 of the second hub 772 of the needle unit 760 so as to connect the needle unit 760 to the prefilled syringe 702. Then, the user takes the needle unit 760 out of the needle unit case 762. While holding the outer cylinder 712, the user punctures the skin of a living body with the needle body 766 and places the needle point intradermally. Next, the user presses the plunger flange 746 toward the leading end with his/her thumb. Accordingly, the pressing portion 740 of the plunger 708 contacts the gasket 706, and the gasket 706 is displaced toward the leading end, which enables injection of the drug inside the inner cylinder 710 into the skin through an inner hole of the needle body 766. After intradermal injection of the drug, the needle body 766 is removed from the skin.

As illustrated in Fig. 1, the package body 12 is made from paper in a cuboid shape. It should be noted that the package body 12 may include a material other than paper such as resin. The package body 12 includes a package base 16 and a cover 18 provided openable with respect to the package base 16. The package base 16 is included in a bottom surface 12a and a side surface 12b of the package body 12. The cover 18 is included in a top surface 12c of the package body 12 and a part of the side surface 12b in the direction of arrow Y1. In the example of Fig. 1, the package body 12 is elongated in the direction of arrow X, the bottom surface 12a is placed in the direction of arrow Z1, and the top surface 12c is placed in the direction of arrow Z2.

The support 14 is formed by vacuum forming or pressure forming of a resin material. Examples of the resin material included in the support 14 include butadienestyrene (BS), acrylonitrile ethylene-propylene-diene styrene (AES), polycarbonate (PC), polypropylene (PP), polyethylene (PE), polystyrene (PS), high impact polystyrene (HIPS), polyethylene terephthalate (PET), and polyvinyl chloride (PVC).

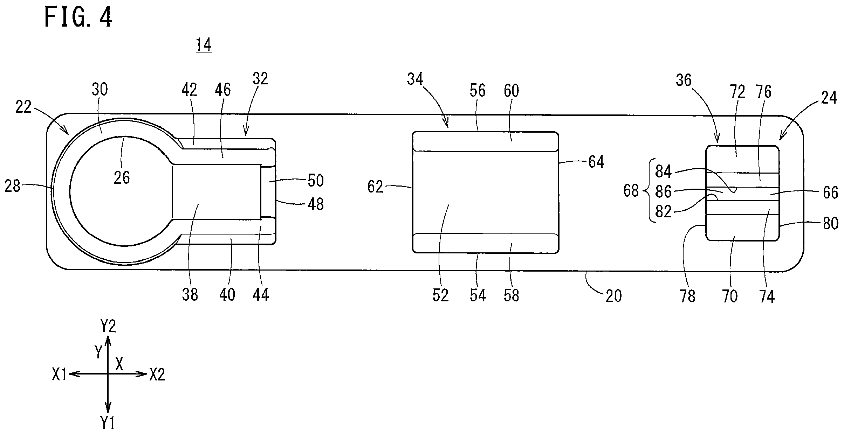

As illustrated in Fig. 1 and Figs. 4 to 6, the support 14 includes a base 20, a needle unit case support 22, and a syringe support 24. The base 20 is a plate-shaped member extended in a longitudinal direction of the package body 12. The needle unit case support 22 supports the needle unit case 762 of the needle package 704. The syringe support 24 supports the prefilled syringe 702. The base 20 is placed on the bottom surface 12a of the package body 12, being substantially equal to the bottom surface 12a in size. In other words, the side surface 12b of the package body 12 regulates displacement of the base 20 in a direction parallel to the bottom surface 12a (in the directions of arrows X and Y). That is, the base 20 holds positions of the needle unit case support 22 and the syringe support 24 inside the package body 12.

The needle unit case support 22 includes an inner periphery 26, an outer periphery 28, and a top portion 30. The inner periphery 26 and the outer periphery 28 project toward the side opposite to the bottom surface 12a (in the direction of arrow Z2) from an end of one end of the base 20 (end in the direction of arrow X1). The top portion 30 connects a projecting end of the inner periphery 26 and that of the outer periphery 28 with each other. The inner periphery 26 is formed in a circular ring shape.

The projecting end of the inner periphery 26 in the direction of arrow X2 is cut out. The outer periphery 28 is formed in a substantial C-shape in a plan view. A projecting length of the needle unit case support 22 is set to about one third of the total length of the needle unit case 762. It should be noted that the projecting length of the needle unit case support 22 may be set to any length.

The syringe support 24 includes a leading end support (leading end regulation unit) 32, an intermediate support (positional shift regulation unit) 34, and a base end support (displacement regulation unit) 36. The leading end support 32 supports a leading end of the prefilled syringe 702. The intermediate support 34 supports an intermediate portion of the syringe body 705. The base end support 36 supports the base end of the syringe body 705.

The leading end support 32 projects from the base 20 in the direction of arrow Z2, being connected to the needle unit case support 22 and extended in the direction of arrow X. The leading end support 32 includes a first curved surface 38, two first side surfaces 40 and 42, two first top portions 44 and 46, and a first end surface 48. The first curved surface 38 is linked to the inner periphery 26. The first side surfaces 40 and 42 are placed on both sides of the first curved surface 38 in the direction of arrow Y. The first top portions 44 and 46 connect the first side surfaces 40 and 42, respectively, with the first curved surface 38. The first end surface 48 is oriented in the direction of arrow X2.

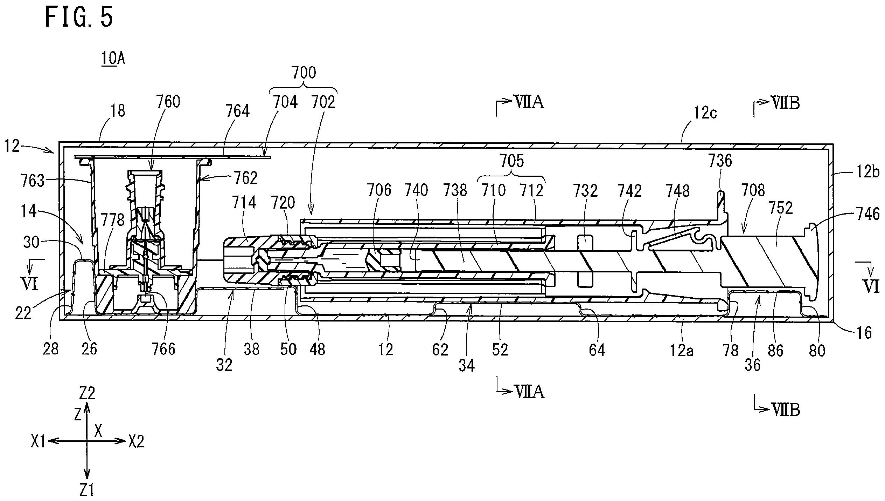

The first curved surface 38 has an arcuate transverse section. A width (dimension in the direction of arrow Y) of the first curved surface 38 is larger than a width (outside diameter) of the cap 714 of the syringe body 705. An end of the first curved surface 38 in the direction of arrow X2 is provided with a curved projection 50 that projects in the direction of arrow Z2. The curved projection 50 is extended over the entire width of the first curved surface 38. An outer surface of the connection 720 of the syringe body 705 comes into contact with the curved projection 50 (see Fig. 5). The first end surface 48 is linked to the first curved surface 38 (curved projection 50), each of the first side surfaces 40 and 42, and each of the first top portions 44 and 46. The two first side surfaces 40 and 42 are linked to the outer periphery 28 and sandwich the first curved surface 38 in the direction of arrow Y. Each of the first top portions 44 and 46 is linked to the top portion 30 of the needle unit case support 22 in a coplanar manner.

The intermediate support 34 projects from the base 20 in the direction of arrow Z2. The intermediate support 34 is spaced from the leading end support 32 in the direction of arrow X2. The intermediate support 34 includes a second curved surface 52, two second side surfaces 54 and 56, two second top portions 58 and 60, and two second end surfaces 62 and 64. The second curved surface 52 has an arcuate (substantially semicircular) transverse section. The second side surfaces 54 and 56 are placed on both sides of the second curved surface 52. The second top portions 58 and 60 connect the second side surfaces 54 and 56, respectively, with the second curved surface 52. The second end surfaces 62 and 64 are oriented in the direction of arrow X.

A width (dimension in the direction of arrow Y) of an end of the second curved surface 52 in the direction of arrow Z2 is formed somewhat smaller than the outside diameter of the outer cylinder 712 (see Fig. 7A). The two second side surfaces 54 and 56 sandwich the second curved surface 52 in the direction of arrow Y. Each of the second side surfaces 54 and 56 is extended in the direction of arrow X. Each of the second end surfaces 62 and 64 is linked to the second curved surface 52, each of the second side surfaces 54 and 56, and each of the second top portions 58 and 60.

The base end support 36 projects from the base 20 in the direction of arrow Z2. The base end support 36 is spaced from the intermediate support 34 in the direction of arrow X2. A length L1 in the direction of arrow X of the base end support 36 is shorter than a length L2 between the plunger flange 746 and the base end of the outer cylinder 712 of the syringe body 705 not in use (see Fig. 6). A difference ΔL between the length L1 and the length L2 is smaller than a space L3 between the gasket 706 and the plunger 708 of the syringe body 705 not in use.

The base end support 36 includes a groove forming unit 68, two third side surfaces 70 and 72, two third top portions 74 and 76, and two third end surfaces 78 and 80. The groove forming unit 68 forms a groove (slit) 66 into which the first protruding portion 752 of the plunger 708 is inserted. The third side surfaces 70 and 72 are placed on both sides of the groove forming unit 68. The third top portions 74 and 76 connect the third side surfaces 70 and 72, respectively, with the groove forming unit 68. The third end surfaces 78 and 80 are oriented in the direction of arrow X.

As illustrated in Fig. 7B, the groove forming unit 68 includes a groove side surface 82, a groove side surface 84, and a groove bottom surface 86. The groove side surface 82 is inclined from the third top portion 74 toward the base 20 in the direction of arrow Y2. The groove side surface 84 is inclined from the third top portion 76 toward the base 20 in the direction of arrow Y1. The groove bottom surface 86 connects the groove side surface 82 and the groove side surface 84 with each other. In other words, a width of the groove 66 is gradually reduced toward the groove bottom surface 86, and a depth of the groove 66 is larger than a projecting length of the first protruding portion 752.

The two third side surfaces 70 and 72 are inclined, approaching each other in the direction of arrow Z2. The third top portion 74 connects the third side surface 70 and the groove side surface 82 with each other. The third top portion 76 connects the third side surface 72 and the groove side surface 84 with each other. Each of the third end surfaces 78 and 80 is linked to the groove forming unit 68, each of the third side surfaces 70 and 72, and each of the third top portions 74 and 76.

In the syringe package 10A with such an arrangement, the needle package 704 is inserted into the inside of the inner periphery 26 of the needle unit case support 22. Specifically, the needle unit case 762 is inserted into the inside of the inner periphery 26. Accordingly, the needle unit case 762 pushes out (elastically deforms) the inner periphery 26, causing action of resilience (elastic force) of the inner periphery26 on the outer surface of the peripheral wall 788 of the needle unit case 762. This action allows the needle unit case support 22 to regulate displacement of the needle package 704 in the directions of arrows X, Y and Z .with respect to the support 14. In other words, the needle package 704 is held by the needle unit case support 22.

On the other hand, the prefilled syringe 702 is attached to the leading end support 32, the intermediate support 34, and the base end support 36. Specifically, the connection 720 of the prefilled syringe 702 is mounted on the first curved surface 38 of the leading end support 32, and the outer cylinder 712 of the prefilled syringe 702 is attached to the second curved surface 52 of the intermediate support 34. Accordingly, the outer cylinder 712 pushes out (elastically deforms) the second curved surface 52, causing action of resilience (elastic force) of the second curved surface 52 on the outer periphery of the outer cylinder 712. This action allows the second curved surface 52 to regulate displacement of the outer cylinder 712 in the directions of arrows X, Y and Z with respect to the support 14. In other words, the outer cylinder 712 is held by the intermediate support 34.

The first end surface 48 and a leading end surface of the outer cylinder 712 face each other in the direction of arrow X. Accordingly, the first end surface 48 regulates displacement of the outer cylinder 712 in the direction of arrow X1 with respect to the plunger 708 (support 14). It should be noted that the cap 714 is not in contact with the first curved surface 38.

The first protruding portion 752 of the plunger 708 of the prefilled syringe 702 is inserted into the groove 66 of the base end support 36. This allows the groove forming unit 68 (groove side surfaces 82 and 84) to regulate displacement of the plunger 708 in the direction of arrow Y with respect to the support 14.

In addition, the third end surface 78 and a base end surface of the outer cylinder 712 face each other in the direction of arrow X. This arrangement regulates displacement of the outer cylinder 712 in the direction of arrow X2 with respect to the plunger 708 (support 14). Furthermore, the third end surface 80 and the plunger flange 746 face each other in the direction of arrow X. This arrangement regulates displacement of the plunger 708 in the direction of arrow X1 with respect to the syringe body 705 (support 14). Still further, the plunger flange 746 and a side surface of the package body 12 in the direction of arrow X2 face each other. This arrangement regulates displacement of the plunger 708 in the direction of arrow X2 with respect to the syringe body 705 (support 14). It should be noted that displacement of the plunger 708 toward the base end with respect to the outer cylinder 712 is also regulated by engagement between the first intermediate flange 742 and the projecting portion 734.

According to this embodiment, the base end support 36 regulates the relative displacement of the outer cylinder 712 and the plunger 708 in the direction in which the gasket 706 and the plunger 708 approach each other. Accordingly, it is possible to simplify the arrangement of the prefilled syringe system 700 and to prevent the plunger 708 from contacting the gasket 706 (the gasket 706 from being pressed by the plunger 708) during transportation of the syringe package 10A.

In addition, the base end support 36 is disposed between the outer cylinder 712 and the plunger flange 746. This arrangement effectively prevents displacement of the outer cylinder 712 toward the base end with respect to the plunger 708 and displacement of the plunger 708 toward the leading end (toward the gasket 706) with respect to the outer cylinder 712.

Furthermore, the base end support 36 is provided with the groove 66 into which the first protruding portion 752 of the plunger 708 is inserted. This arrangement prevents separation of the base end support 36 from between the outer cylinder 712 and the plunger flange 746 due to vibration during transportation of the syringe package 10A.

Still further, the intermediate support 34 contacts the outer cylinder 712 and regulates displacement of the outer cylinder 712 in the direction intersecting with the axis. This arrangement further prevents separation of the base end support 36 from between the outer cylinder 712 and the plunger flange 746 due to vibration during transportation of the syringe package 10A.

Still further, the outer cylinder 712 (the intermediate portion of the syringe body 705) is locked with the intermediate support 34. This arrangement further prevents separation of the base end support 36 from between the outer cylinder 712 and the plunger flange 746 due to vibration during transportation of the syringe package 10A.

According to this embodiment, the leading end support 32 regulates displacement of the outer cylinder 712 toward the leading end. This arrangement further prevents a positional shift of the syringe body 705. In addition, the support 14 includes the needle unit case support 22. This arrangement supports the needle package 704 and the prefilled syringe 702 individually inside the package body 12.

According to this embodiment, the needle unit case support 22, the leading end support 32, the intermediate support 34, and the base end support 36 are provided in an integrated manner with respect to the base 20. This arrangement prevents displacement of the needle unit case support 22, the leading end support 32, the intermediate support 34, and the base end support 36 inside the package body 12.

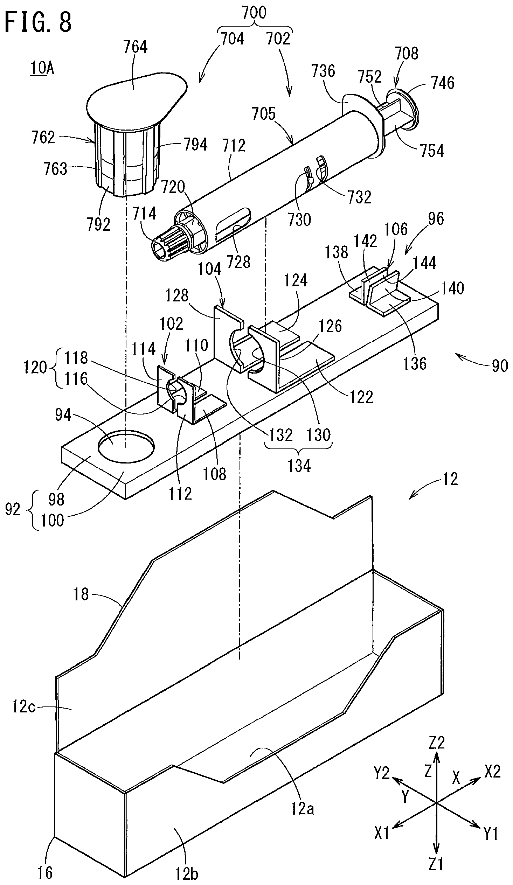

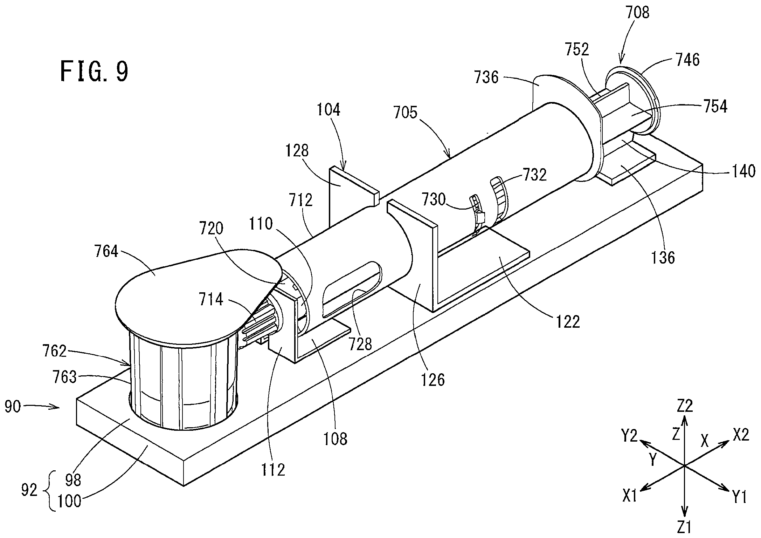

This embodiment is not limited to the above arrangement. For example, the syringe package 10A may be provided with a support 90 illustrated in Figs. 8 and 9 instead of the support 14. As illustrated in Figs. 8 and 9, the support 90 is made from paper, including a base 92 placed on a bottom surface of the package body 12, and a needle unit case support 94 and a syringe support 96 provided on the base 92.

The base 92 includes a rectangular base body 98 and a leg 100. The base body 98 is substantially equal to the bottom surface 12a of the package body 12 in size. The leg 100 is extended from an outer edge of the base body 98. The needle unit case support 94 is a hole formed at an end of one end of the base body 98 (end in the direction of arrow X1).

The syringe support 96 includes a leading end support (leading end regulation unit) 102, an intermediate support (positional shift regulation unit) 104, and a base end support (displacement regulation unit) 106. The leading end support 102 supports the leading end of the syringe body 705. The intermediate support 104 supports the intermediate portion of the syringe body 705. The base end support 106 supports the base end of the syringe body 705.

The leading end support 102 includes two first fixed portions 108 and 110, and two first extended portions 112 and 114. The first fixed portions 108 and 110 are fixed to the base body 98. The first extended portions 112 and 114 are extended from the first fixed portions 108 and 110, respectively, in the direction of arrow Z2. The two first extended portions 112 and 114 are spaced from each other in a width direction (the direction of arrow Y) of the base 92. The space between the first extended portions 112 and 114 is smaller than a width of the connection 720. An arcuate first cut 116 is formed in the first extended portion 112, and an arcuate first cut 118 is formed in the first extended portion 114. The two first cuts 116 and 118 communicate with each other to form one circular first hole 120. Through the first hole 120, the connection 720 of the prefilled syringe 702 is inserted. The first extended portions 112 and 114 regulate displacement of the connection 720 in the directions of arrows Y and Z while the connection 720 is inserted into the first hole 120. A diameter of the first hole 120 is larger than an outside diameter of the connection 720 and smaller than the outside diameter of the outer cylinder 712.

The intermediate support 104 includes two second fixed portions 122 and 124, and two second extended portions 126 and 128. The second fixed portions 122 and 124 are fixed to the base body 98. The second extended portions 126 and 128 are extended from the second fixed portions 122 and 124, respectively, in the direction of arrow Z2. The two second extended portions 126 and 128 are spaced from each other in the width direction (the direction of arrow Y) of the base 92. The space between the second extended portions 126 and 128 is smaller than the outside diameter of the outer cylinder 712. An arcuate second cut 130 is formed in the second extended portion 126, and an arcuate second cut 132 is formed in the second extended portion 128. The two second cuts 130 and 132 communicate with each other to form one circular second hole 134. Through this second hole 134, the outer cylinder 712 of the prefilled syringe 702 is inserted. The second extended portions 126 and 128 regulate displacement of the outer cylinder 712 in the directions of arrows Y and Z while the outer cylinder 712 is inserted into the second hole 134. The second hole 134 has a diameter slightly larger than the outside diameter of the outer cylinder 712.

The base end support 106 includes two third fixed portions 136 and 138, and two third extended portions 140 and 142. The third fixed portions 136 and 138 are fixed to the base body 98. The third extended portions 140 and 142 are extended from the third fixed portions 136 and 138, respectively, in the direction of arrow Z2. Each of the third extended portions 140 and 142 is extended in an extension direction (the direction of arrow X) of the base body 98. The two third extended portions 140 and 142 are spaced from each other in the width direction (the direction of arrow Y) of the base 92. The space between the third extended portions 140 and 142 gradually increases in the direction of arrow Z2. In other words, a gap between the third extended portions 140 and 142 that face each other functions as a groove 144 into which the first protruding portion 752 of the plunger 708 of the prefilled syringe 702 is inserted.

In such a syringe package 10A provided with the support 90 according to the modification, the needle unit case 762 of the needle package 704 is inserted into the needle unit case support 94. This allows the needle unit case support 94 to regulate displacement of the needle package 704 in the directions of arrows X and Y with respect to the support 90. Furthermore, the first extended portions 112 and 114 and the leading end surface of the outer cylinder 712 face each other in the direction of arrow X. This arrangement allows the first extended portions 112 and 114 to regulate displacement of the outer cylinder 712 in the direction of arrow X1 with respect to the plunger 708 (support 90).

The prefilled syringe 702 is attached to the leading end support 102, the intermediate support 104, and the base end support 106. Specifically, the connection 720 of the prefilled syringe 702 is inserted into the first hole 120 of the leading end support 102. This allows the first extended portions 112 and 114 included in the first hole 120 to regulate displacement of the connection 720 in the directions of arrows Y and Z with respect to the support 90.

The outer cylinder 712 of the prefilled syringe 702 is inserted into the second hole 134 of the intermediate support 104. This allows the second extended portions 126 and 128 included in the second hole 134 to regulate displacement of the outer cylinder 712 in the directions of arrows Y and Z with respect to the support 90.

The first protruding portion 752 of the plunger 708 of the prefilled syringe 702 is inserted into the groove 144 of the base end support 106. This allows the third extended portions 140 and 142 included in the groove 144 to regulate displacement of the plunger 708 in the direction of arrow Y with respect to the support 90. In addition, the third extended portions 140 and 142 and the base end surface of the outer cylinder 712 face each other in the direction of arrow X. This arrangement regulates displacement of the outer cylinder 712 in the direction of arrow X2 with respect to the plunger 708 (support 90). Furthermore, the third extended portions 140 and 142 and the plunger flange 746 face each other in the direction of arrow X. This arrangement regulates displacement of the plunger 708 in the direction of arrow X1 with respect to the syringe body 705 (support 90).

In this manner, the support 90 according to the modification also shows effects similar to those shown by the support 14.

### (Second Embodiment)

Next, a syringe package 10B according to a second embodiment of the present invention will be described. In the syringe package 10B according to the second embodiment, the same components as those in the syringe package 10A according to the first embodiment are denoted by the same reference numerals, and detailed description thereof will be omitted. This also applies to a syringe package 10C according to a third embodiment which is to be described later.

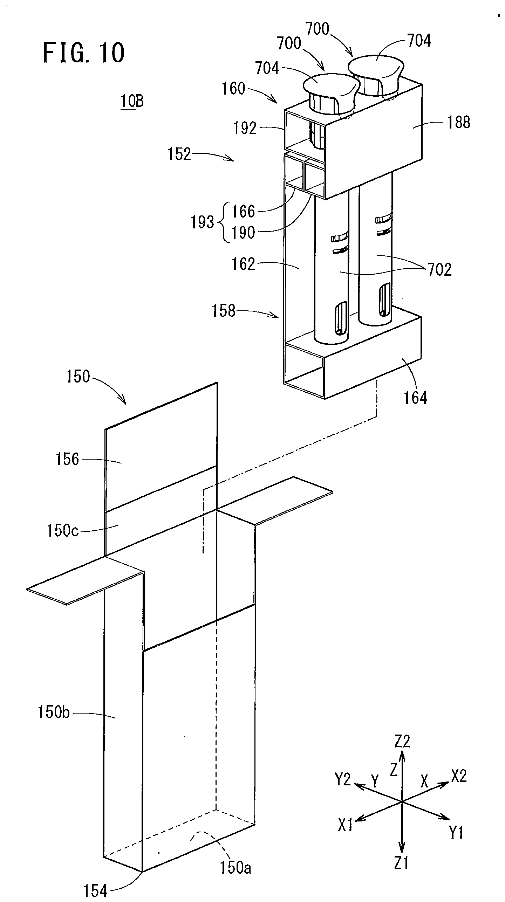

As illustrated in Fig. 10, the syringe package 10B according to this embodiment includes a box-shaped package body 150 and a support 152. The package body 150 houses two sets of prefilled syringes 702 and needle packages 704. The support 152 is provided inside the package body 150. In other words, the syringe package 10B houses two prefilled syringe systems 700 together. Each prefilled syringe system 700 is arranged similarly to the prefilled syringe system 700 according to the first embodiment.

The package body 150 is made from paper in a cuboid shape. It should be noted that the package body 150 may include a material other than paper such as resin. Specifically, the package body 150 includes a package base 154 and a cover 156 that is openable and joined to the package base 154. The package base 154 is included in a bottom surface 150a and a side surface 150b of the package body 150. The cover 156 is included in a top surface 150c of the package body 150 and a part of the side surface 150b in the direction of arrow Y1. In the example of Fig. 10, the package body 150 is elongated in the direction of arrow Z, the bottom surface 150a is placed in the direction of arrow Z1, and the top surface 150c is placed in the direction of arrow Z2.

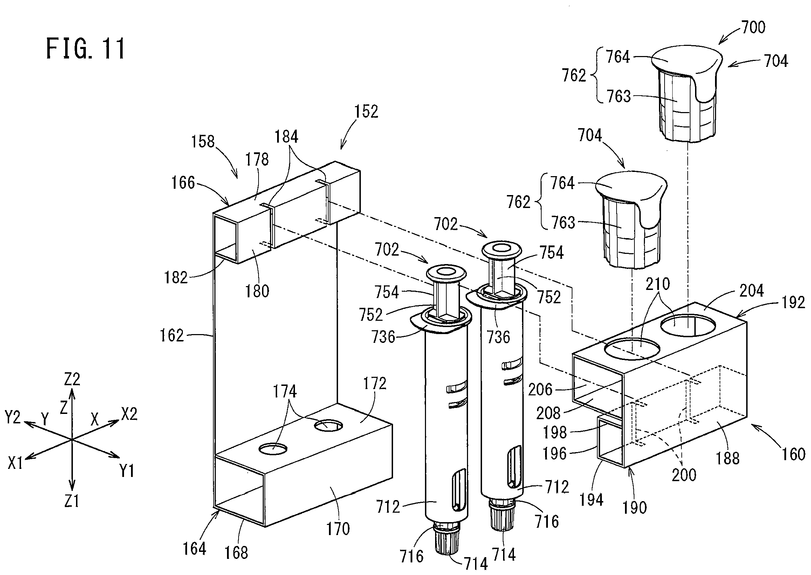

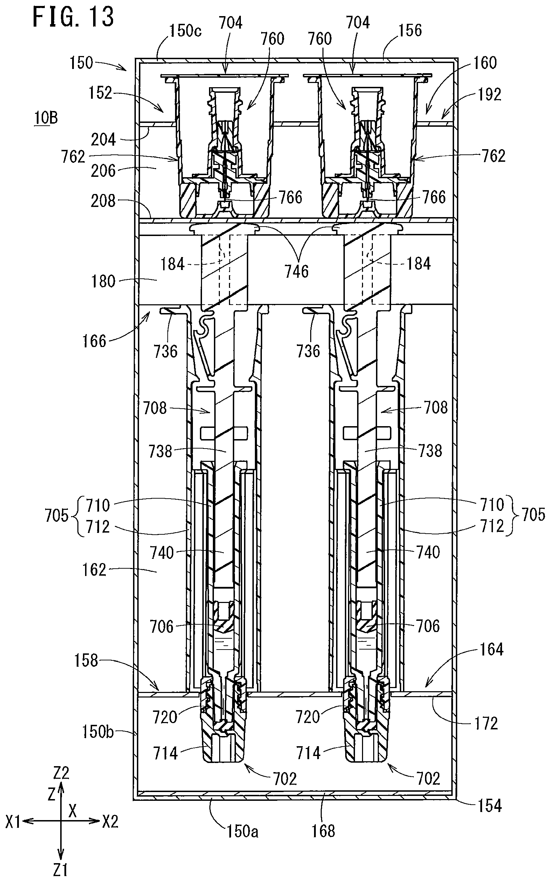

As illustrated in Figs. 10 to 14, the support 152 includes a first support member 158 and a second support member 160. The first support member 158 and the second support member 160 are assembled, for example, by folding paper. The first support member 158 includes a rectangular first base 162, a leading end support (leading end regulation unit) 164, and a first base end support 166. The first base 162 is extended in an extension direction (the direction of arrow Z) of the package body 150. The leading end support 164 supports a leading end of the prefilled syringe 702, and the first base end support 166 supports a base end of the prefilled syringe 702.

A width (dimension in the direction of arrow X) of the first base 162 is substantially equal to a width of the side surface 150b of the package body 150 in the direction of arrow Y2. The leading end support 164 is formed in a substantial U-shape in a side view, being provided at one end (end in the direction of arrow Z1) of the first base 162. Specifically, the leading end support 164 includes a first wall 168, a second wall 170, and a third wall 172. The first wall 168 projects from one end of the first base 162 in the direction of arrow Y1. The second wall 170 projects from a projecting end of the first wall 168 in the direction of arrow Z2. The third wall 172 is extended from a projecting end of the second wall 170 in the direction of arrow Y2. A projecting end face of the third wall 172 is fixed to the first base 162 with a fixing member (adhesive or the like). It should be noted that the third wall 172 may not be fixed to the first base 162.

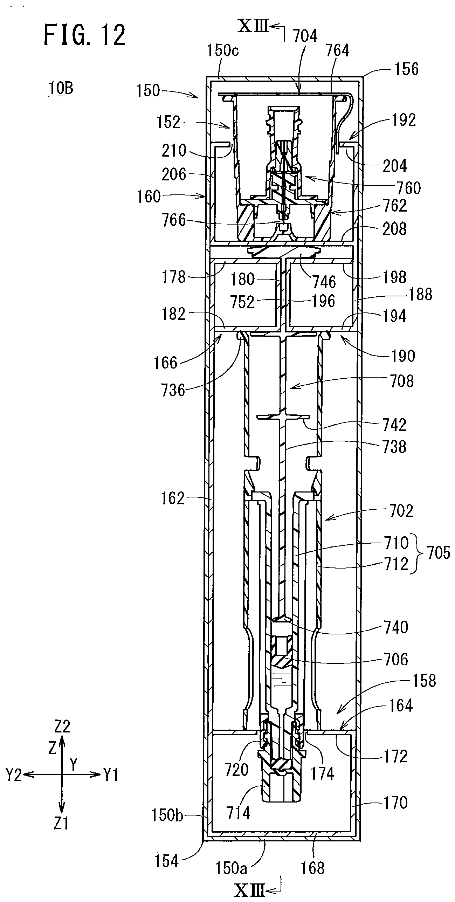

An interval between the first wall 168 and the third wall 172 that face each other is slightly longer than a distance from a leading end of the outer cylinder 712 of the prefilled syringe 702 to a leading end of the cap 714 (see Fig. 12). The third wall 172 is provided with two syringe support holes 174 through which the connection 720 of the prefilled syringe 702 is inserted. The syringe support holes 174 are arranged side by side in the direction of arrow X. Each syringe support hole 174 has a diameter smaller than an outside diameter of the outer cylinder 712.

The first base end support 166 is formed in a substantial U-shape in a side view, being provided at the other end (end in the direction of arrow Z2) of the first base 162. Specifically, the first base end support 166 includes a fourth wall 178, a fifth wall 180, and a sixth wall 182. The fourth wall 178 projects from the other end of the first base 162 in the direction of arrow Y1. The fifth wall 180 projects from a projecting end of the fourth wall 178 in the direction of arrow Z1. The sixth wall 182 projects from a projecting end of the fifth wall 180 in the direction of arrow Y2.

Projecting lengths of the fourth wall 178 and the sixth wall 182 are shorter than projecting lengths of the first wall 168 and the third wall 172. The fifth wall 180 is provided with two first grooves (slits) 184 into which a first protruding portion 752 of the plunger 708 is inserted. The first grooves 184 are formed over the entire length of the fifth wall 180 in the direction of arrow Z. The first grooves 184 are spaced from each other in the direction of arrow X. A projecting end face of the sixth wall 182 is fixed to the first base 162 with a fixing member (adhesive or the like). It should be noted that the sixth wall 182 may not be fixed to the first base 162. An interval between the sixth wall 182 and the fourth wall 178 is substantially equal to the entire length of the outer cylinder 712.

The second support member 160 includes a second base 188, a second base end support 190 that supports the base end of the prefilled syringe 702, and a needle support 192 that supports a needle package 704. The second base end support 190 is formed in a substantial U-shape in a side view, facing the first base end support 166 in the direction of arrow Y. In this embodiment, the first base end support 166 and the second base end support 190 are included in one base end support (displacement regulation unit) 193.

Specifically, the second base end support 190 includes a seventh wall 194, an eighth wall 196, and a ninth wall 198. The seventh wall 194 projects in the direction of arrow Y2 from an end of the second base 188 in the direction of arrow Z2. The eighth wall 196 projects from a projecting end of the seventh wall 194 in the direction of arrow Z1. The ninth wall 198 projects from a projecting end of the eighth wall 196 in the direction of arrow Y1.

Projecting lengths of the seventh wall 194 and the ninth wall 198 are substantially equal to the projecting lengths of the fourth wall 178 and the sixth wall 182 (see Fig. 12). A predetermined gap is formed between the fifth wall 180 and the eighth wall 196. The eighth wall 196 is provided with two second grooves (slits) 200 into which the first protruding portion 752 of the plunger 708 is inserted. The second grooves 200 are formed over the entire length of the eighth wall 196 in the direction of arrow Z. The second grooves 200 are spaced from each other in the direction of arrow X. In addition, each second groove 200 faces each first groove 184. A projecting end face of the ninth wall 198 is fixed to the second base 188 with a fixing member (adhesive or the like). It should be noted that the ninth wall 198 may not be fixed to the second base 188.

The needle support 192 is formed in a substantial U-shape in a side view, being placed on the first base end support 166 and the second base end support 190 in the opposite direction of the leading end support 164 (in the direction of arrow Z2). Specifically, the needle support 192 includes a tenth wall 204, an eleventh wall 206, and a twelfth wall 208. The tenth wall 204 projects in the direction of Y2 from an end of the second base 188 in the direction of arrow Z2. The eleventh wall 206 projects from a projecting end of the tenth wall 204 in the direction of arrow Z1. The twelfth wall 208 projects from a projecting end of the eleventh wall 206 in the direction of arrow Y1.

Projecting lengths of the tenth wall 204 and the twelfth wall 208 are substantially equal to the projecting lengths of the first wall 168 and the third wall 172. The tenth wall 204 is provided with two needle unit case supports 210 each functioning as a hole through which the needle unit case 762 of the needle package 704 is inserted. The needle unit case supports 210 are arranged side by side in the direction of arrow X. An interval between the twelfth wall 208 and the fourth wall 178 and an interval between the twelfth wall 208 and the ninth wall 198 are formed to be slightly larger than a thickness of the plunger flange 746.

To the syringe package 10B with such an arrangement, two prefilled syringes 702 are attached side by side in the direction of arrow X and two needle packages 704 are attached side by side in the direction of arrow X. In other words, one prefilled syringe 702 is attached to the syringe support hole 174, the first groove 184, and the second groove 200 placed in the direction of arrow X1, while the other prefilled syringe 702 is attached to the syringe support hole 174, the first groove 184, and the second groove 200 placed in the direction of arrow X2. Furthermore, one needle package 704 is attached to the needle unit case support 210 placed in the direction of arrow X1, while the other needle package 704 is attached to the needle unit case support 210 placed in the direction of arrow X2.

Specifically, the connection 720 of the prefilled syringe 702 is inserted into the syringe support hole 174. This allows the third wall 172 to regulate displacement of the outer cylinder 712 in the direction of arrow X1 with respect to the plunger 708 (the support 152). One second protruding portion 754 of the plunger 708 of the prefilled syringe 702 is inserted into the first groove 184, while the other second protruding portion 754 of the plunger 708 is inserted into the second groove 200. A first protruding portion 752 is disposed in a gap between the fifth wall 180 and the eighth wall 196. This regulates displacement of the plunger 708 in the directions of arrows X and Y with respect to the support 152.

A base end (outer cylinder flange 736) of the outer cylinder 712 of the prefilled syringe 702 is placed on the sixth wall 182 and the seventh wall 194 in the direction of arrow Z1. In other words, a base end surface of the outer cylinder 712 of the prefilled syringe 702 and the sixth wall 182 and the seventh wall 194 face each other in the direction of arrow Z. This allows the sixth wall 182 and the seventh wall 194 to regulate displacement of the outer cylinder 712 in the direction of arrow Z2 with respect to the plunger 708 (support 152).

The plunger flange 746 is placed between the fourth wall 178 and the twelfth wall 208 and between the ninth wall 198 and the twelfth wall 208. This arrangement regulates displacement of the plunger 708 toward the leading end (in the direction of arrow Z1) with respect to the syringe body 705 (support 152).

Furthermore, the needle unit case 762 contacts the twelfth wall 208 while being inserted into the needle unit case support 210. This arrangement allows the tenth wall 204 to regulate displacement of the needle package 704 in the directions of arrows X and Y with respect to the support 152. It should be noted that displacement of the needle package 704 in the direction of arrow Z2 with respect to the support 152 is regulated by the top surface 150c of the package body 150.

The syringe package 10B according to this embodiment shows effects similar to those shown by the syringe package 10A according to the first embodiment. This embodiment is not limited to the above arrangement. The syringe package 10B may house one or three or more prefilled syringe systems 700 together.

### (Third Embodiment)

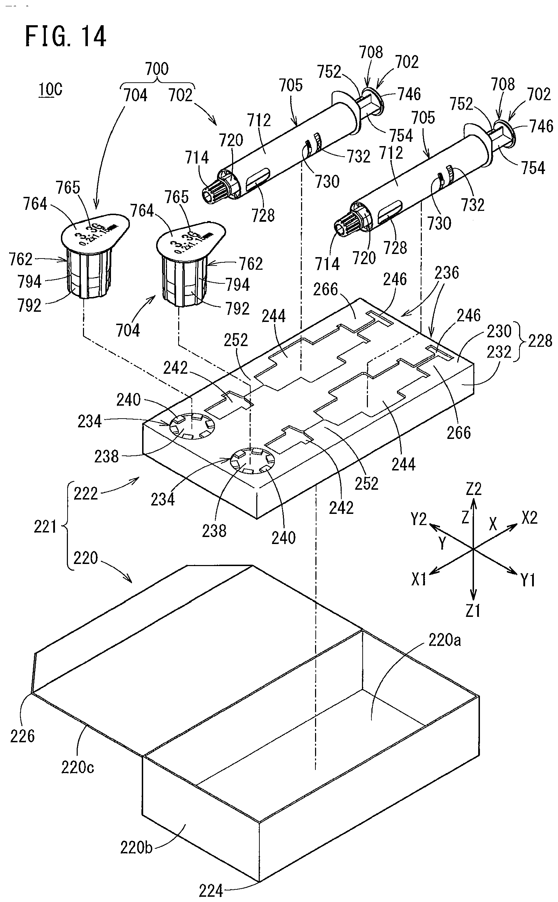

Next, a syringe package 10C according to a third embodiment of the present invention will be described. As illustrated in Fig. 14, an outer surface of a sealing film 764 in a prefilled syringe system 700 of the syringe package 10C is provided with a displayed information 765 visible and associated with a needle unit 760. Examples of this information include a gauge (inside diameter), an outside diameter, and a length (projecting length from a needle hub 768) of a needle body 766.

The syringe package 10C includes a package unit 221. The package unit 221 includes a box-shaped package body 220 and a support 222. The package body 220 houses two sets of prefilled syringes 702 and needle packages 704. The support 222 is provided inside the package body 220. In other words, the syringe package 10C houses two prefilled syringe systems 700 together. Each prefilled syringe system 700 is arranged similarly to the prefilled syringe system 700 according to the first embodiment.

The package body 220 is made from paper in a cuboid shape. It should be noted that the package body 220 may include a material other than paper such as resin. Specifically, the package body 220 includes a package base 224 and a cover 226 that is openable and joined to the package base 224. The package base 224 is included in a bottom surface 220a and a side surface 220b of the package body 220. The cover 226 is included in a top surface 220c of the package body 220 and a part of the side surface 220b in the direction of arrow Y1. In the example of Fig. 14, the package body 220 is elongated in the direction of arrow X, the bottom surface 220a is placed in the direction of arrow Z1, and the top surface 220c is placed in the direction of arrow Z2.

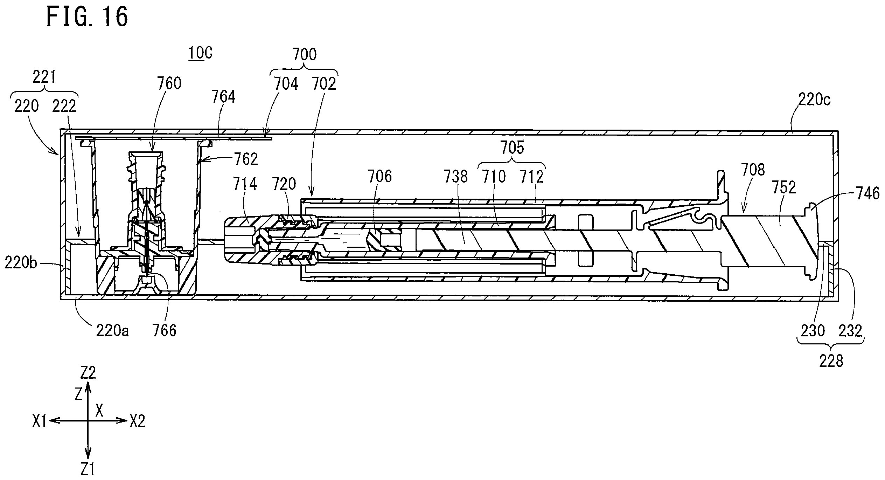

As illustrated in Figs. 14 to 16, the support 222 includes a base 228 made from paper. The base 228 includes a rectangular base body 230 and a leg 232 extended from an outer edge of the base body 230. The base body 230 is substantially equal to the bottom surface 220a of the package body 220 in size. In other words, the leg 232 contacts each side surface 220b of the package body 220. The base body 230 is provided with two needle unit case supports 234 each of which supports the needle package 704 and two syringe supports 236 each of which supports the prefilled syringe 702.

Each of the needle unit case supports 234 includes a needle unit case support hole 238 formed at one end of the base body 230 in a longitudinal direction (end in the direction of arrow X1). The two needle unit case support holes 238 are placed at a predetermined interval in a width direction (the direction of arrow Y) of the base body 230. A wall surface included in each needle unit case support hole 238 is provided with a plurality of protrusions 240 at equal intervals in a circumferential direction. The protrusions 240 are extended toward the center (radially inward) of the needle unit case support hole 238. These protrusions 240 are to be inserted into recesses 792 of a needle unit case 762. Accordingly, the number and positions of the protrusions 240 correspond to the number of the recesses 792 of the needle unit case 762.

Each syringe support 236 includes a leading end support 242, an intermediate support 244, and a base end support 246. The leading end support 242 is a hole into which a leading end of the prefilled syringe 702 is inserted. The intermediate support 244 is a hole into which an intermediate portion of the prefilled syringe 702 is inserted. The base end support 246 is a hole into which a plunger 708 of the prefilled syringe 702 is inserted.

Each leading end support 242 includes a first hole 248 into which a cap 714 of the prefilled syringe 702 is inserted and a second hole 250 which is linked to the first hole 248 in the direction of arrow X2 and into which a leading end portion of the outer cylinder 712 is inserted. The first hole 248 is narrower (a width in the direction of arrow Y is narrower) than the second hole 250.

Between the leading end support 242 and the intermediate support 244, two first locking units (positional shift regulation units) 252 are provided. The first locking units 252 are respectively inserted into first opening portions 728 of the prefilled syringe 702. In other words, while the prefilled syringe 702 is not attached to the support 222, the first locking units 252 are brought into contact with each other and partition the leading end support 242 and the intermediate support 244. Each first locking unit 252 locks a syringe body 705.

The intermediate support 244 includes a third hole 254, a fourth hole 256, a fifth hole 258, and a sixth hole 260. Into the third hole 254, inserted is a part of a leading end of the outer cylinder 712 which is close to a base end from the first opening portions 728. The fourth hole 256 is wide and linked to the third hole 254 in the direction of arrow X2. The fifth hole 258 is linked to the fourth hole 256 in the direction of arrow X2, functioning as a hole into which the base end of the outer cylinder 712 is inserted. The sixth hole 260 is linked to the fifth hole 258 in the direction of arrow X2, functioning as a hole into which an outer cylinder flange 736 is inserted. Widths of the third hole 254 and the fifth hole 258 are substantially equal to a width of the second hole 250. The fourth hole 256 has a size large enough to allow a user to insert his/her finger into a space outside the outer cylinder 712 while the outer cylinder 712 is inserted into the fourth hole 256. A width of the sixth hole 260 is wider than the width of the fifth hole 258.

The base end support 246 includes a seventh hole (groove) 262 linked to the sixth hole 260 in the direction of arrow X2, and an eighth hole 264 linked to the seventh hole 262 in the direction of arrow X2. Into the seventh hole 262, a first protruding portion 752 of the plunger 708 is inserted, and into the eighth hole 264, a plunger flange 746 is inserted. A width of the seventh hole 262 is slightly larger than a thickness of the first protruding portion 752. In other words, between the sixth hole 260 and the seventh hole 262, provided are two second locking units (displacement regulation units) 266 that cover a second protruding portion 754 from the direction of arrow Z2 while the prefilled syringe 702 is attached to the support 222. The seventh hole 262 is formed between the second locking units 266.

To the syringe package 10C with such an arrangement, two needle packages 704 are attached side by side in the direction of arrow Y and two syringes are attached side by side in the direction of arrow Y. Specifically, the needle unit case 762 of the needle package 704 is inserted into the needle unit case support hole 238. This arrangement allows a wall surface included in the needle unit case support hole 238 to regulate displacement of the needle package 704 in the directions of arrows X and Y with respect to the support 222.

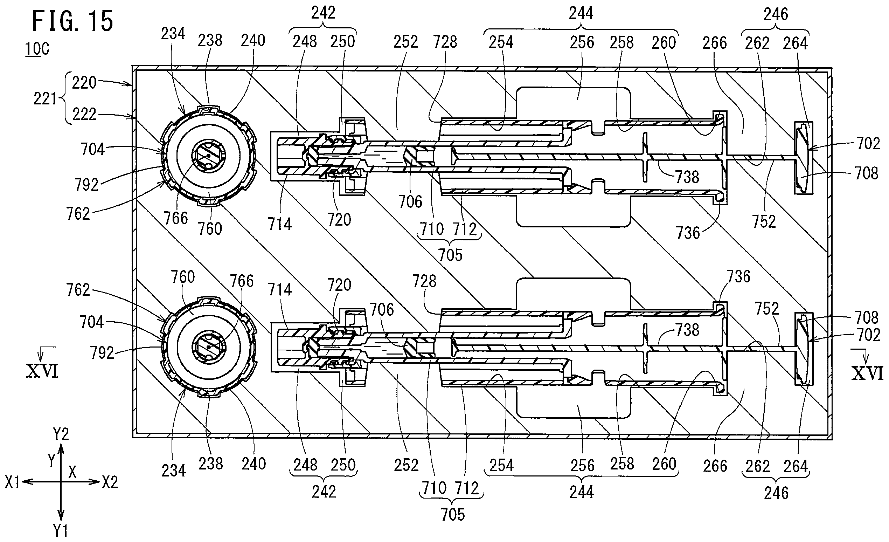

In this state, as illustrated in Fig. 15, the protrusions 240 are inserted into the recesses 792 of the needle unit case 762. This arrangement allows the protrusions 240 to regulate rotation of the needle package 704 around the axis. Accordingly, it is possible to hold the information visible and displayed on the outer surface of the sealing film 764 in a fixed orientation. Therefore, when opening the package body 220, it is easy to visually check the displayed information 765 associated with the needle unit 760.

Into the first opening portions 728 of the prefilled syringe 702, the first locking units (leading end regulation units) 252 are inserted. Accordingly, the first locking units 252 regulate displacement of the outer cylinder 712 in the directions of arrows Y and Z with respect to the support 222. In addition, the first locking units 252 regulate displacement of the outer cylinder 712 in the direction of arrow X with respect to the plunger 708 (the support 222).

Into the seventh hole 262, the first protruding portion 752 of the plunger 708 of the prefilled syringe 702 is inserted. Accordingly, the second locking units 266 regulate displacement of the plunger 708 in the direction of arrow Y with respect to the support 222. Furthermore, the second locking units 266 cover the second protruding portion 754 from the direction of arrow Z2. This arrangement regulates displacement of the plunger 708 in the direction of arrow Z2 with respect to the support 222.

A base end surface of the outer cylinder 712 and the second locking units 266 face each other in the direction of arrow X. This arrangement regulates displacement of the outer cylinder 712 in the direction of arrow X2 with respect to the plunger 708 (support 222). The plunger flange 746 and the second locking units 266 face each other in the direction of arrow X. This arrangement regulates displacement of the plunger 708 in the direction of arrow X1 with respect to the syringe body 705 (support 222).

The syringe package 10C according to this embodiment shows effects similar to those shown by the syringe package 10A according to the first embodiment. In addition, the syringe package 10C shows effects similar to those shown by the syringe package 10G according to a first reference example which is to be described later.

This embodiment is not limited to the above arrangement. The syringe package 10C may house one or three or more prefilled syringe systems 700 together.

In the syringe packages 10A to 10C, when not in use, the gasket 706 and the plunger 708 may not be connected to each other but in contact with each other, and when in use, the gasket 706 and the plunger 708 may be connected. Furthermore, in the syringe packages 10A to 10C, when not in use, the needle unit 760 may be connected to the connection 720 of the prefilled syringe 702.

### (Fourth Embodiment)

Next, a syringe package 10D according to a fourth embodiment will be described. In the syringe package 10D according to the fourth embodiment, the same components as those in the syringe package 10C according to the third embodiment are denoted by the same reference numerals, and detailed description thereof will be omitted.

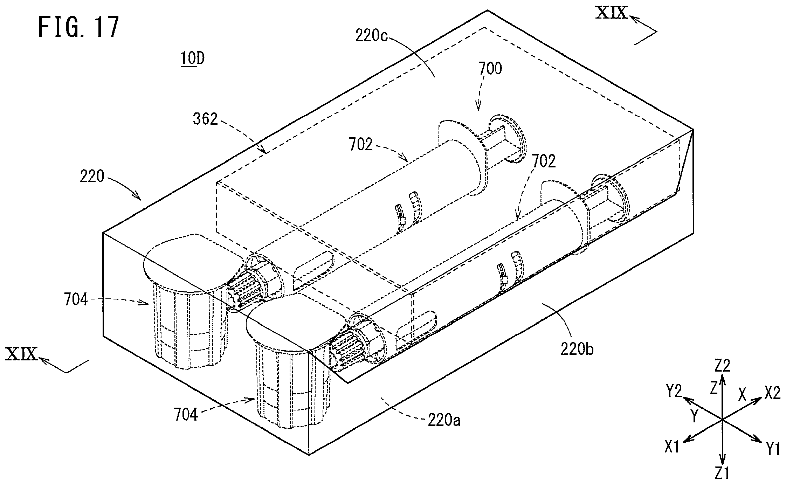

As illustrated in Figs. 17 to 19, the syringe package 10D includes a package insert case 362 that houses a package insert 360. The package insert case 362 is housed inside a package body 220 together with a support 222.

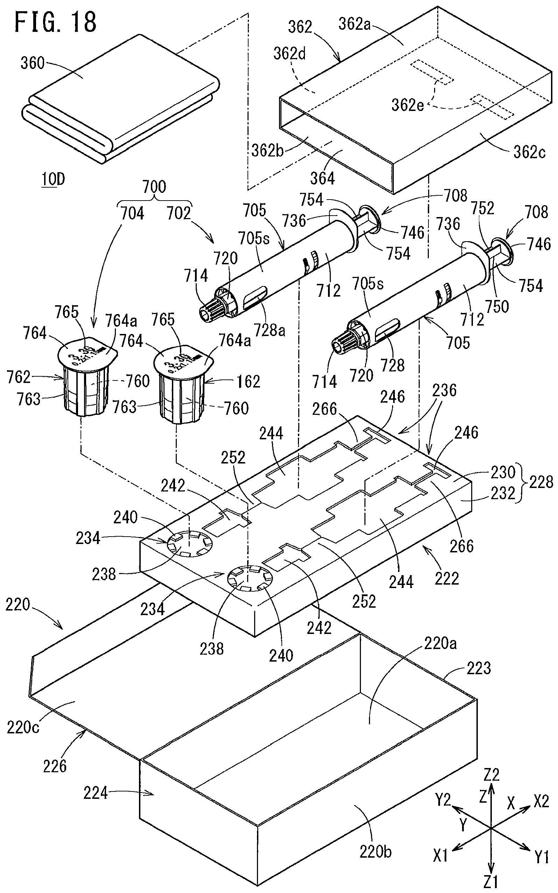

As illustrated in Fig. 18, the package body 220 includes a housing base (housing body) 224 and a cover 226. The housing base 224 is provided with a fetch opening 223 in an upper portion of the package body 220. The cover 226 closes the fetch opening 223. A bottom surface (bottom wall) 220a and a side surface (peripheral wall) 220b are included in the housing base 224. A top surface (top wall) 220c is included in the cover 226. The cover 226 is formed in an integrated manner with the housing base 224, being linked to an upper edge of a wall included in one side of the side surface 220b. It should be noted that the cover 226 may be a member that is separable from the housing base 224.

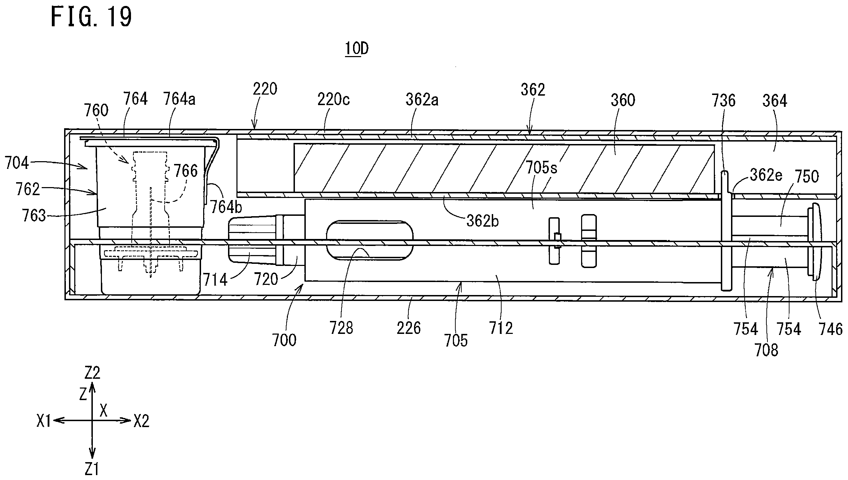

A needle unit case 762 is disposed closer to the top surface 220c than (an outer surface 705s of) a syringe body 705, projecting closer to the cover 226 than the syringe body 705. Specifically, in a housed state illustrated in Fig. 19, an upper surface of the needle unit case 762 (a surface 764a of a sealing film 764) is higher than the outer surface 705s of the syringe body 705.

Being detachable, the sealing film 764 is disposed on the needle unit case 762 to close an opening portion of a case body 763. The sealing film 764 is provided with a tab 764b which a user pinches when opening the needle unit case 762. In this embodiment, as illustrated in Fig. 19, the tab 764b is bent along a side surface of the case body 763 on the side close to the package insert case 362, and the tab 764b is detachably fixed to the side surface. As illustrated in Fig. 18, the surface 764a of the sealing film 764 may be provided with displayed information (such as size and expiration date) 765 associated with a needle unit 760. A part or all of the displayed information 765 may be provided on a surface of the tab 764b.

In Fig. 18, the package insert case 362 is a paper member formed in a flat cuboid shape, including a housing 364 (housing space) that houses the package insert 360. It should be noted that the package insert case 362 may include a material other than paper such as resin. The housing 364 penetrates the package insert case 362 in a direction (the direction of arrow X) perpendicular to a thickness direction (the direction of arrow Z) of the package body 220.

A length (dimension in the direction of arrow X) of the package insert case 362 is set to a length slightly shorter than a distance between the needle unit case 762 and a side portion in the direction of arrow X2 inside the package body 220. A width (dimension in the direction of arrow Y) of the package insert case 362 is set to be substantially equal to an internal width (dimension in the direction of arrow Y) of the housing base 224 of the package body 220. A thickness (dimension in the direction of arrow Z) of the package insert case 362 is set to be substantially equal to a distance between the outer surface 705s of the syringe body 705 housed inside the package body 220 and the top surface 220c of the package body 220.

As illustrated in Fig. 18, the package insert case 362 includes a pair of base walls 362a and 362b spaced from and facing each other in the thickness direction of the package body 220, and a pair of side walls 362c and 362d that connects the pair of base walls 362a and 362b. The housing 364 is a space surrounded by the pair of base walls 362a and 362b and the pair of side walls 362c and 362d.

In Fig. 19, the package insert case 362 is disposed between the syringe body 705 and the top surface 220c, being in contact with the syringe body 705 to prevent movement of the prefilled syringe 702 inside the package body 220. Specifically, one (upper) base wall 362a is in contact with an inner surface of the cover 226 of the package body 220. The other (lower) base wall 362b is placed lower than a surface of the needle unit case 762 that faces the top surface 220c (lower than the surface 764a of the sealing film 764), being in contact with the outer surface 705s of the syringe body 705.

As illustrated in Fig. 18, the package insert case 362 is provided with a flange opening 362e. Specifically, the flange opening 362e is provided in the lower base wall 362b. While the prefilled syringe 702 is housed, an outer cylinder flange 736 of the prefilled syringe 702 is inserted into the flange opening 362e. In this embodiment, since the syringe package 10D houses two prefilled syringes 702, the syringe package 10D is provided with two flange openings 362e. The flange openings 362e communicate with the housing 364.

The package insert 360 is an instruction manual that describes how to use a prefilled syringe system 700. The package insert 360 may include a plurality of documents. Therefore, the package insert 360 may include documents other than the instruction manual. As illustrated in Fig. 18, the package insert 360 is, for example, folded and housed in the package insert case 362.

In the related art, there is known a syringe system which is provided with a syringe and a needle unit and in which the needle unit is separated from the syringe in an initial state (at the time of product offering) and the needle unit is connected to the syringe when in use (for example, JP 5756793 B2)

This type of syringe system is offered to a user as a product while the syringe and a needle unit case that houses the needle unit are housed inside a box-like package. Typically, in the package, a package insert such as an instruction manual is stored in addition to the syringe system or the product. It is desirable that such a package be made compact and that the syringe inside the package be held at a predetermined position.

In the syringe package 10D according to the fourth embodiment, the needle unit case 762 projects closer, than the syringe body 705, to at least one wall (the top surface 220c) of the walls (the top surface 220c and the bottom surface 220a) facing each other in the package body 220. The package insert case 362 is disposed between the syringe body 705 and one wall (the top surface 220c), being in contact with the outer surface 705s of the syringe body 705.

Accordingly, the package insert case 362 is disposed in a space which is between the syringe body 705 and one wall (top surface 220c) of the package body 220 and which otherwise becomes a dead space. In addition, the package insert case 362 prevents movement of the prefilled syringe 702 inside the package body 220. The effective use of the dead space makes it possible to make the syringe package 10D compact and to preferably hold a position of the prefilled syringe 702 inside the package body 220.

In the fourth embodiment, a base end portion of the syringe body 705 is provided with the outer cylinder flange 736 projecting in a direction perpendicular to the axis of the syringe body 705, and the package insert case 362 is provided with the flange opening 362e into which the outer cylinder flange 736 is inserted. This makes it is possible to enlarge the package insert case 362 while avoiding interference between the package insert case 362 and the outer cylinder flange 736, which leads to a further effective use of the dead space.

Furthermore, in the fourth embodiment, the package body 220 includes the housing base 224 that is provided with the fetch opening 223 and the cover 226 that closes the fetch opening 223 and one wall (the top surface 220c) of the package body 220 is included in the cover 226. Accordingly, when the cover 226 is opened, the package insert case 362 is exposed, which makes it easier for a user to find the package insert 360.

Still further, in the fourth embodiment, the needle unit case 762 includes the case body 763 and the sealing film 764 that seals the case body 763, and the sealing film 764 is provided with the tab 764b which the user pinches when opening the needle unit case 762. The tab 764b is bent along the side surface of the case body 763 on the side close to the package insert case 362, and the tab 764b is detachably fixed to the side surface (see Fig. 19). This arrangement prevents the sealing film 764 from being peeled from the case body 763 (from opening the needle unit case 762) due to vibration during, for example, transportation. In addition, when the package insert case 362 is taken out, it is possible to prevent the sealing film 764 from being peeled from the package insert case 362.

Unlike the above arrangement, the tab 764b may be overlapped with an outer surface of the package insert case 362 that faces the top surface 220c. In other words, the tab 764b may be sandwiched between the top surface 220c and the package insert case 362 (the base wall 362a). According to this arrangement, even when the displayed information 765 (see Fig. 18) on the needle unit 760 is provided on the surface of the tab 764b, the displayed information 765 is not hidden by the package insert case 362. Accordingly, it is possible for the user to quickly understand the information associated with the needle unit 760 when opening the package body 220.

In the fourth embodiment, other components same as or similar to those in the third embodiment offer functions and effects similar to those obtained in the third embodiment.

### (Fifth Embodiment)

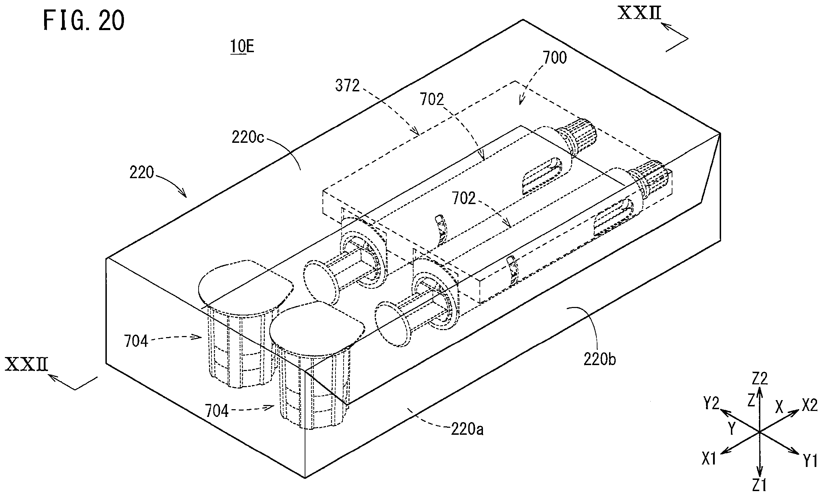

A syringe package 10E according to a fifth embodiment illustrated in Figs. 20 to 22 is provided with a prefilled syringe 702, a needle package 704, a support 370 that supports the prefilled syringe 702 and the needle package 704, a package insert case 372 that houses a package insert 360, and a package body 220 that houses the prefilled syringe 702, the needle package 704, the support 370, and the package insert case 372. The package body 220 according to the fifth embodiment is arranged similarly to the package body 220 according to the fourth embodiment.

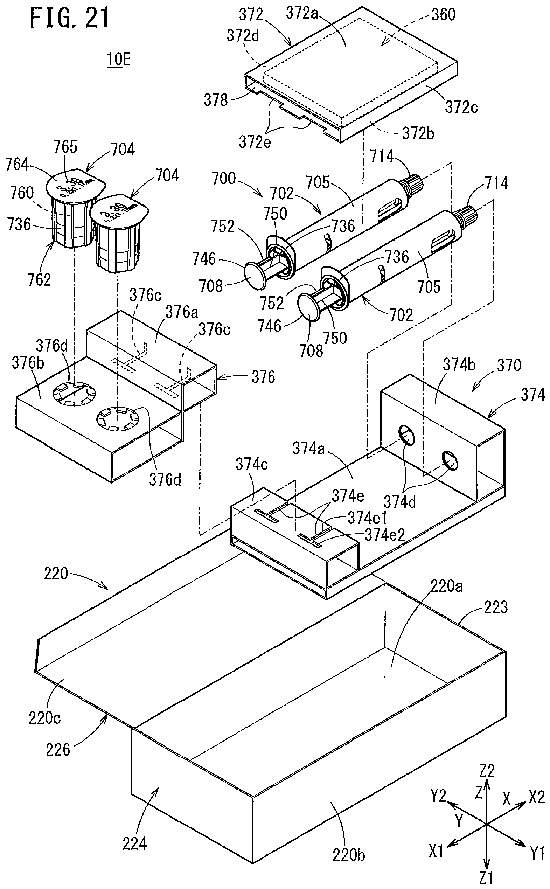

In Fig. 21, the support 370 includes a first support member 374 and a second support member 376. The first support member 374 and the second support member 376 are assembled, for example, by folding paper. The first support member 374 includes a rectangular base 374a, a leading end support (leading end regulation unit) 374b, and a first base end support 374c. The base 374a is extended in an extension direction (the direction of arrow X) of the package body 220. The leading end support 374b supports a leading end of the prefilled syringe 702. The first base end support 374c supports a base end of the prefilled syringe 702.

A width (dimension in the direction of arrow Y) of the base 374a is substantially equal to a distance between side portions facing each other in the direction of arrow Y in the package body 220. The leading end support 374b projects upward from one end (end in the direction of arrow X2) of the base 374a. A surface of the leading end support 374b close to the first base end support 374c is provided with a syringe support hole 374d into which a connection 720 of the prefilled syringe 702 is inserted. In this embodiment, since the syringe package 10E houses two prefilled syringes 702, the syringe package 10E is provided with two syringe support holes 374d at an interval in the direction of arrow Y.

The first base end support 374c projects upward from the other end (end in the direction of arrow X1) of the base 374a. A height of the first base end support 374c projecting from the base 374a is lower than a height of the leading end support 374b projecting from the base 374a. The first base end support 374c is provided with a T-shaped first holding groove 374e.

The first holding groove 374e includes a first slit 374e1 extended along the extension direction (the direction of arrow X) of the package body 220 and a second slit 374e2 extended in a direction (the direction of arrow Y) perpendicular to the first slit 374e1. While the prefilled syringe 702 is housed, a first protruding portion 752 of a plunger 708 is inserted into the first slit 374e1, and a plunger flange 746 is inserted into the second slit 374e2. In the fifth embodiment, since two prefilled syringes 702 are housed, two first holding grooves 374e are provided at an interval in the direction of arrow Y.

The second support member 376 includes a second base end support 376a that supports the base end of the prefilled syringe 702 and a needle unit case support 376b that supports the needle package 704. The second base end support 376a faces the first base end support 374c above the first base end support 374c. While the prefilled syringe 702 is housed, a base end support (displacement regulation unit) 375 including the first base end support 374c and the second base end support 376a sandwiches a base end portion of the plunger 708.

The second base end support 376a is provided with a T-shaped second holding groove 376c. While the prefilled syringe 702 is housed, the base end portion of the plunger 708 (the first protruding portion 752 of a shaft 738 and the plunger flange 746) is inserted into the second holding groove 376c. In the fifth embodiment, since two prefilled syringes 702 are housed, two second holding grooves 376c are provided at an interval in the direction of arrow Y.

In this manner, in the package body 220, a leading end portion (connection 720) of the prefilled syringe 702 is inserted into the syringe support hole 374d provided in the leading end support 374b of the first support member 374. Furthermore, a base end portion of the prefilled syringe 702 (the base end portion of the plunger 708) is inserted into the first holding groove 374e provided in the first base end support 374c and into the second holding groove 376c provided in the second base end support 376a. Accordingly, a plurality of prefilled syringes 702 inside the package body 220 is held at predetermined positions by the support 370 provided with the first support member 374 and the second support member 376.

An upper surface of the needle unit case support 376b is provided with a needle unit case support hole 376d into which a needle unit case 762 of the needle package 704 is inserted. In the fifth embodiment, since the two needle packages 704 are housed, two needle unit case support holes 376d are provided at an interval in the direction of arrow Y.

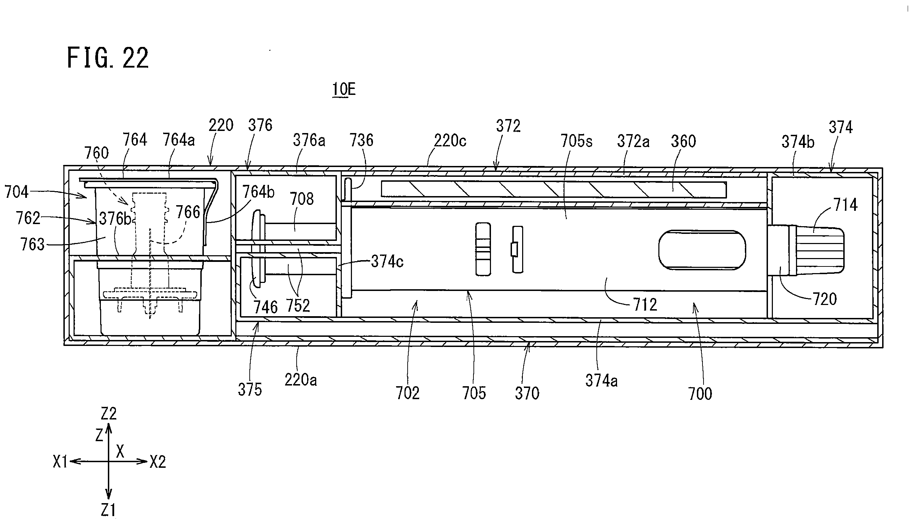

As illustrated in Fig. 22, the upper surface of the needle unit case support 376b is placed lower than an upper surface of the second base end support 376a. The needle unit case support 376b supports the needle unit case 762 so that a needle body 766 of a needle unit 760 inside the needle unit case 762 is along a thickness direction (the direction of arrow Z) of the package body 220. In other words, while the needle unit case 762 is housed inside the package body 220, the needle body 766 is along the thickness direction of the package body 220.

Being disposed closer to a top surface 220c than (an outer surface 705s of) a syringe body 705, the needle unit case 762 projects closer to the top surface 220c than the syringe body 705. Specifically, in the fifth embodiment, in a housed state illustrated in Fig. 22, an upper surface (a surface 764a of a sealing film 764) of the needle unit case 762 is higher than the outer surface 705s of the syringe body 705.

In Fig. 21, the package insert case 372 is a paper member formed in a flat cuboid shape, including a housing 378 (housing space) that houses the package insert 360. It should be noted that the package insert case 372 may include a material other than paper such as resin. The housing 378 penetrates the package insert case 372 in a direction ( direction of arrow X) perpendicular to the thickness direction ( direction of arrow Z) of the package body 220.

A length (dimension in the direction of arrow X) of the package insert case 372 is set to be substantially equal to a distance between the leading end support 374b and the second base end support 376a inside the package body 220. A width (dimension in the direction of arrow Y) of the package insert case 372 is set to be substantially equal to an internal width (dimension in the direction of arrow Y) of a housing body 224 of the package body 220. A thickness (dimension in the direction of arrow Z) of the package insert case 372 is set to be substantially equal to a distance between the outer surface 705s of the syringe body 705 housed inside the package body 220 and the top surface 220c (cover 226) of the package body 220.

The package insert case 372 includes a pair of base walls 372a and 372b spaced from and facing each other in the thickness direction of the package body 220, and a pair of side walls 372c and 372d that connects the pair of base walls 372a and 372b. The housing 378 is a space surrounded by the pair of base walls 372a and 372b and the pair of side walls 372c and 372d.

In Fig. 22, the package insert case 372 is disposed between the syringe body 705 and the top surface 220c, being in contact with the syringe body 705 to prevent movement of the prefilled syringe 702 inside the package body 220. Specifically, one (upper) base wall 372a is in contact with an inner surface of the top surface 220c of the package body 220. The other (lower) base wall 372b is placed lower than a surface of the needle unit case 762 that faces the top surface 220c (lower than the surface 764a of the sealing film 764), being in contact with the outer surface 705s of the syringe body 705.

As illustrated in Fig. 21, the package insert case 372 is provided with a flange opening 372e. Specifically, in the fifth embodiment, the flange opening 372e is formed into a cut, being provided at an end of the lower base wall 372b in the direction of arrow X1. An outer cylinder flange 736 of the prefilled syringe 702 is inserted into the flange opening 372e. In this embodiment, since the syringe package 10E houses two prefilled syringes 702, the syringe package 10E is provided with two flange openings 372e. The flange openings 372e communicate with the housing 378.

As described above, in the syringe package 10E according to the fifth embodiment, the needle unit case 762 projects closer, than the syringe body 705, to at least one wall (the top surface 220c) of the walls (the top surface 220c and the bottom surface 220a) facing each other in the package body 220. The package insert case 372 is disposed between the syringe body 705 and one wall (the top surface 220c), being in contact with the outer surface 705s of the syringe body 705.

Accordingly, the package insert case 372 is disposed in a space which is between the syringe body 705 and one wall (top surface 220c) of the package body 220 and which otherwise becomes a dead space. In addition, the package insert case 372 prevents movement of the prefilled syringe 702 inside the package body 220. Even in the fifth embodiment, the effective use of the dead space makes it possible to make the syringe package 10E compact and to preferably hold a position of the prefilled syringe 702 inside the package body 220.

In the fifth embodiment, other components same as or similar to those in the third and fourth embodiments offer functions and effects similar to those obtained in the third and fourth embodiments.

### (Sixth Embodiment)

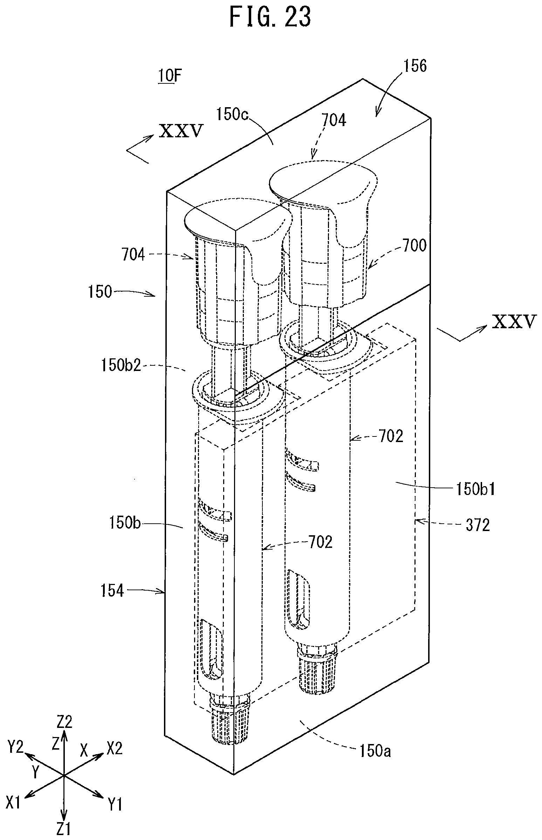

A syringe package 10F according to a sixth embodiment illustrated in Figs. 23 to 25 is provided with a prefilled syringe 702, a needle package 704, a support 152 that supports the prefilled syringe 702 and the needle package 704, a package insert case 372 that houses a package insert 360, and a package body 150 that houses the prefilled syringe 702, the needle package 704, the support 152, and the package insert case 372. In this syringe package body 10F, an extension direction (longitudinal direction) of the package body 150 is along the vertical direction (the direction of arrow Z).

The package body 150 is a paper member formed in a flat cuboid shape. It should be noted that the package body 150 may include a material other than paper such as resin. As illustrated in Fig. 23, the package body 150 includes a bottom surface (bottom wall) 150a, a side surface (peripheral wall) 150b, and a top surface (top wall) 150c. The side surface 150b includes a rectangular front wall 150b1 and a rear wall 150b2 included in the walls spaced from each other in a thickness direction (the direction of arrow Y) of the package body 150. Both the front wall 150b1 and the rear wall 150b2 are formed in a flat plate shape and are parallel to each other when the package body 150 is unopened.

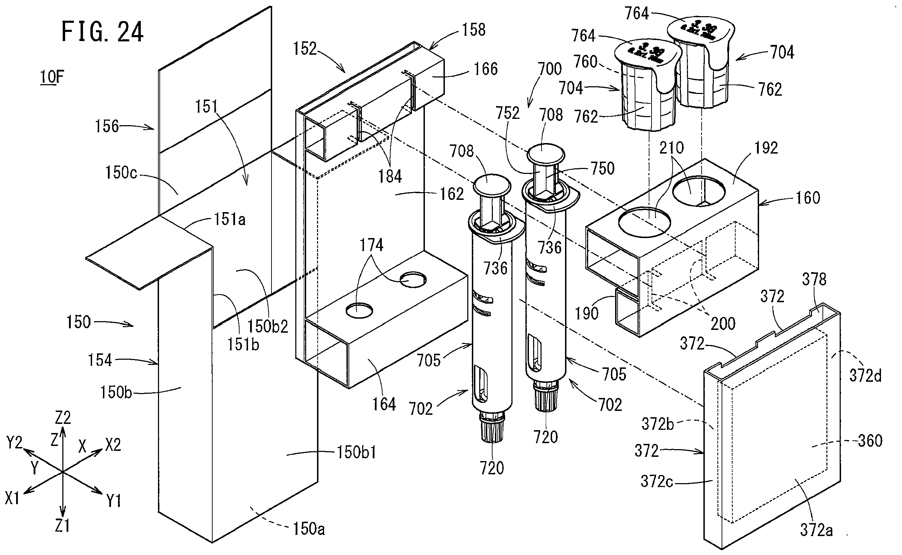

As illustrated in Fig. 24, the package body 150 includes a package base (housing body) 154 which is provided with a fetch opening 151 and a cover 156 which closes the fetch opening 151. The fetch opening 151 includes a first opening 151a which opens upward (in the direction of arrow Z2) and a second opening 151b which opens in the thickness direction of the package body 150 (in the direction of arrow Y1).

The bottom surface 150a and the side surface 150b (except for a portion that covers the second opening 151b) are included in the package base 154. Furthermore, in the top surface 150c and the front wall 150b1, those portions that cover the second opening 151b are included in the cover 156. The cover 156 is formed in an integrated manner with the package base 154, being linked to an upper edge of the rear wall 150b2 included in one side of the side surface 150b. It should be noted that the cover 156 may be a member that is separable from the package base 154.

The support 152 is arranged similarly to the support 152 according to the second embodiment.

In the support 152 inside the package body 150, a leading end portion (connection 720) of the prefilled syringe 702 is inserted into a syringe support hole 174 provided in a leading end support 164 of a first support member 158. Furthermore, a base end portion of the prefilled syringe 702 (a base end portion of a plunger 708) is inserted into a first groove 184 provided in a first base end support 166 and into a second groove 200 provided in a second base end support 190. Accordingly, a plurality of prefilled syringes 702 inside the package body 150 is held at predetermined positions by the support 152 provided with the first support member 158 and the second support member 160.

In addition, a plunger flange 746 is engaged with an upper surface of the first base end support 166 and is held between the second base end support 190 and a needle support 192. This arrangement holds axial positions of the plunger 708 with respect to a syringe body 705 and a gasket 706 (see Fig. 12).

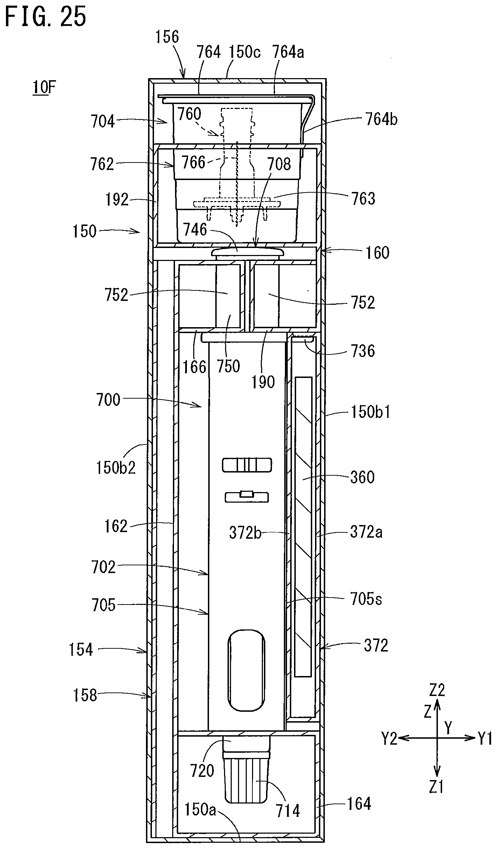

An upper surface of the needle support 192 is provided with a needle unit case support 210 into which a needle unit case 762 of the needle package 704 is inserted. In the sixth embodiment, since two needle packages 704 are housed, two needle unit case supports 210 are provided at an interval in the direction of arrow X. As illustrated in Fig. 25, in the package body 150, the upper surface of the needle support 192 is spaced from the top surface 150c and faces the top surface 150c.

The needle support 192 supports the needle unit case 762 so that a needle body 766 of a needle unit 760 inside the needle unit case 762 is along an extension direction (the direction of arrow Z) of the package body 150. In other words, while the needle unit case 762 is housed inside the package body 150, the needle body 766 is along the extension direction of the package body 150 and an extension direction of the prefilled syringe 702. The needle unit case 762 is disposed closer to the front wall 150b1 than (an outer surface 705s of) the syringe body 705, projecting closer to the front wall 150b1 than the syringe body 705.

The package insert case 372 according to the sixth embodiment illustrated in Fig. 24 is configured similarly to the package insert case 372 according to the fifth embodiment illustrated in Fig. 22, including a pair of base walls 372a and 372b and a pair of side walls 372c and 372d. It should be noted that, in Fig. 24, the housing 378 penetrates the package insert case 372 along the extension direction (the direction of arrow Z) of the package body 150.

A length (dimension in the direction of arrow Z) of the package insert case 372 is set to be substantially equal to a distance between the leading end support 164 and the second base end support 190 inside the package body 150. A width (dimension in the direction of arrow X) of the package insert case 372 is set to be substantially equal to an internal width (dimension in the direction of arrow X) of the package base 154 of the package body 150. A thickness (dimension in the direction of arrow Y) of the package insert case 372 is set to be substantially equal to a distance between the outer surface 705s of the syringe body 705 housed inside the package body 150 and the front wall 150b1 of the package body 150.

In Fig. 25, the package insert case 372 is disposed between the syringe body 705 and the front wall 150b1, being in contact with the syringe body 705 to prevent movement of the prefilled syringe 702 inside the package body 150. Specifically, one base wall 372a is in contact with an inner surface of the front wall 150b1 of the package body 150. The other base wall 372b is placed closer to the rear wall 150b2 than the section closest to the front wall 150b1 of the needle unit case 762, being in contact with the outer surface 705s of the syringe body 705.

As described above, in the syringe package 10F according to the sixth embodiment, the needle unit case 762 projects closer, than the syringe body 705, to at least one wall (the front wall 150b1) of the walls (the front wall 150b1 and the rear wall 150b2) facing each other in the package body 150. The package insert case 372 is disposed between the syringe body 705 and one wall (the front wall 150b1), being in contact with the outer surface 705s of the syringe body 705.

Accordingly, the package insert case 372 is disposed in a space which is between the syringe body 705 and one wall (front wall 150b1) of the package body 150 and which otherwise becomes a dead space. In addition, the package insert case 372 prevents movement of the prefilled syringe 702 inside the package body 150. Even in the sixth embodiment, the effective use of the dead space makes it possible to make the syringe package 10F compact and to preferably hold a position of the prefilled syringe 702 inside the package body 150.

In the sixth embodiment, other components same as or similar to those in the second and fifth embodiments offer functions and effects similar to those obtained in the second and fifth embodiments.

### (Reference Example)

Next, a syringe package 10G according to a reference example will be described. In the syringe package 10G according to the reference example, the same components as those in the syringe package 10A according to the first embodiment are denoted by the same reference numerals, and detailed description thereof will be omitted.

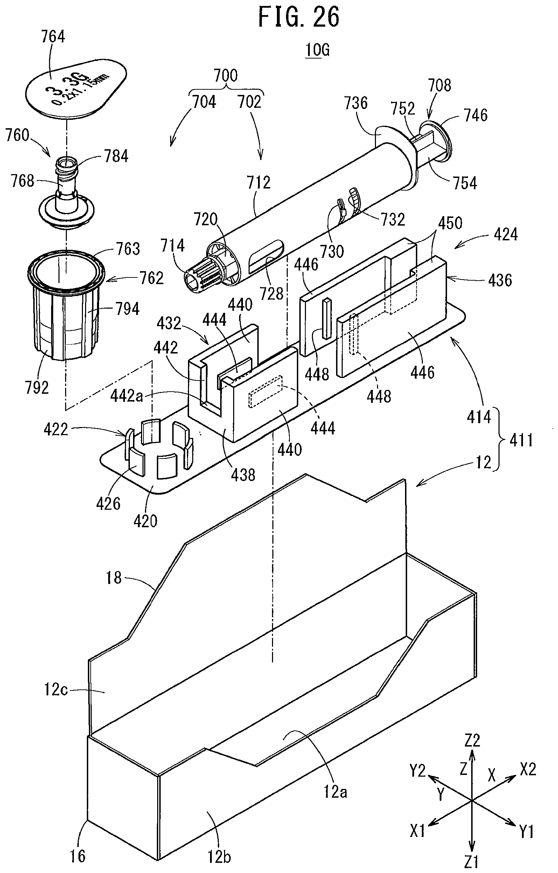

As illustrated in Fig. 26, the syringe package 10G according to the reference example includes a prefilled syringe system 700 and a package unit 411. An outer surface of a film (sealing film) 764 in the prefilled syringe system 700 displays information visible and associated with a needle unit 760. Examples of this information include a gauge (inside diameter), an outside diameter, and a length (projecting length from a needle hub 768) of a needle body 766.

The package unit 411 includes a box-shaped package body 12 and a support 414 which is provided inside the package body 12 and supports the prefilled syringe system 700.

The support 414 is formed by vacuum forming or pressure forming of a resin material. Examples of the resin material included in the support 414 are similar to the examples of the resin material included in the support 14.

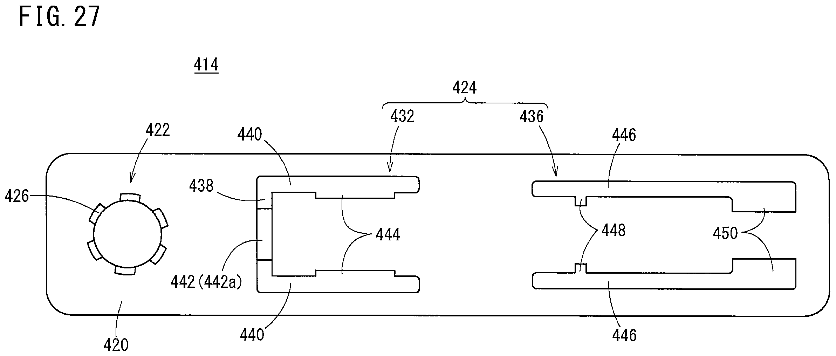

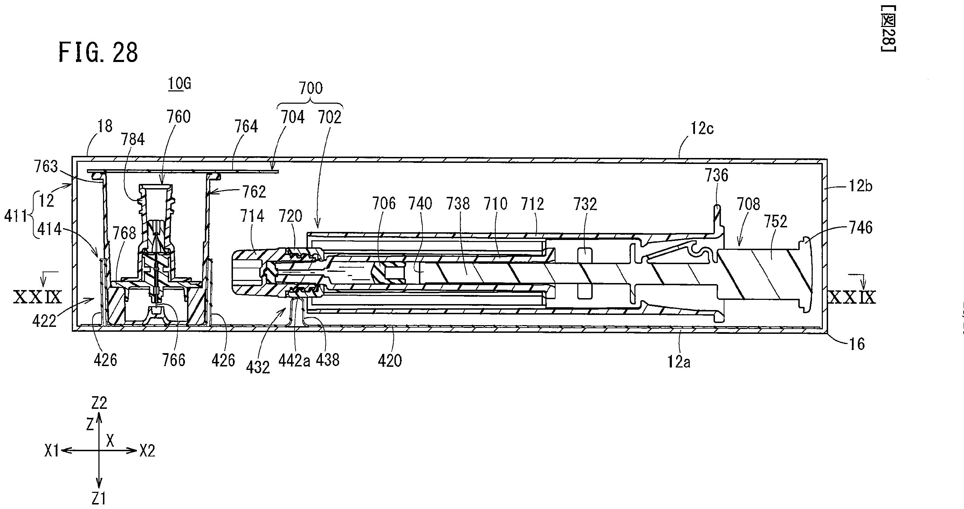

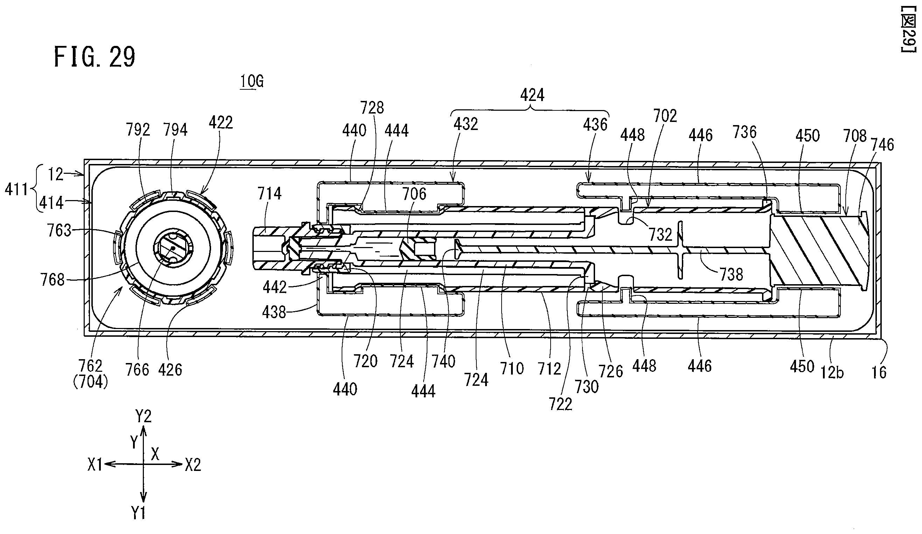

As illustrated in Figs. 26 to 29, the support 414 includes a base 420, a needle unit case support 422, and a syringe support 424. The base 420 is a plate-shaped member extended in a longitudinal direction of the package body 12. The needle unit case support 422 supports a needle unit case 762 of a needle package 704. The syringe support 424 supports a prefilled syringe 702. The base 420 is placed on a bottom surface 12a of the package body 12, being substantially equal to the bottom surface 12a in size. In other words, displacement of the base 420 in a direction parallel to the bottom surface 12a (the directions of arrows X and Y) is regulated by a side surface 12b of the package body 12. That is, the base 420 holds positions of the needle unit case support 422 and the syringe support 424 inside the package body 12.

The needle unit case support 422 has a plurality of projecting portions (second locking units) 426 projecting toward the side opposite to the bottom surface 12a (in the direction of arrow Z2) from an end of one end of the base 420 (end in the direction of arrow X1). Each projecting portion 426 has a shape corresponding to each recess 792 of the needle unit case 762. The projecting portions 426 are respectively inserted into the recesses 792. The plurality of projecting portions 426 is provided at equal intervals in a circumferential direction of the needle unit case 762, corresponding to the recesses 792.

The syringe support 424 includes a leading end support 432 that supports a leading end of the prefilled syringe 702 and a base end support 436 that supports a base end of the prefilled syringe 702.

The leading end support 432 projects from the base 420 in the direction of arrow Z2, being formed in a substantial U-shape in a plan view. The leading end support 432 includes a first wall 438 and two second walls 440. The first wall 438 is extended in a width direction (the direction of arrow Y) of the base 420. The second walls 440 are extended in the direction of arrow X2 from both ends of the first wall 438 in the direction of arrow Y. The first wall 438 is provided with a U-shaped recessed portion 442 in which a connection 720 of the prefilled syringe 702 is provided. A height of a wall surface 442a of the recessed portion 442 in the direction of arrow Z2 (an interval between the base 420 and the wall surface 442a) is set to such a degree that allows a cap 714 not to contact the base 420. In the second walls 440, surfaces that face each other are provided with first projections (leading end projections) 444 projecting in a direction in which the first projections 444 approach each other. The first projections 444 are inserted into first opening portions 728 of an outer cylinder 712. Each of the first projections 444 is extended in the direction of arrow X, corresponding to the shape of the first opening portions 728.

The base end support 436 projects from the base 420 in the direction of arrow Z2. The base end support 436 includes two third walls 446. The third walls 446 are extended in the direction of arrow X, facing each other while being spaced from each other in the direction of arrow Y. In the third walls 446, surfaces close to the direction of arrow X1 are provided with second projections (base end projections) 448 projecting in a direction in which the second projections 448 approach each other. The second projections 448 are inserted into third opening portions 732 of the outer cylinder 712. Each of the second projections 448 is extended in the direction of arrow Z, corresponding to the shape of each third opening portion 732. An end portion of each third wall 446 is provided with a fourth wall 450. Each of the fourth walls 450 is extended in a direction in which both fourth walls 450 approach each other. An interval between the fourth walls 450 that face each other is smaller than the outer shape of a plunger flange 746 and has a size that allows insertion of a shaft 738 of a plunger 708.

In the syringe package 10G with such an arrangement, the needle package 704 is inserted into the inside of the projecting portions 426 of the needle unit case support 422. Specifically, the plurality of projecting portions 426 is inserted into the recesses 792 of the needle unit case 762. This allows the needle unit case support 422 to regulate displacement of the needle unit case 762 in the directions of arrows X and Y with respect to the support 414. Furthermore, such an arrangement regulates rotation of the needle unit case 762 in the circumferential direction (about the axis) with respect to the support 414.

On the other hand, the prefilled syringe 702 is attached to the leading end support 432 and the base end support 436. Specifically, the connection 720 of the prefilled syringe 702 is inserted into the recessed portion 442 of the leading end support 432, and the outer cylinder 712 of the prefilled syringe 702 is inserted into a space between the second walls 440 facing each other and a space between the third walls 446 facing each other, and the plunger 708 is inserted into a space between the fourth walls 450 facing each other.

Accordingly, the first wall 438 regulates displacement of the outer cylinder 712 in the direction of arrow X1 with respect to the support 414 (plunger 708) of the outer cylinder 712, the second walls 440 and the third walls 446 regulate displacement of the outer cylinder 712 in the direction of arrow Y with respect to the support 414, and the fourth walls 450 regulate displacement of the outer cylinder 712 in the direction of arrow X2 with respect to the support 414 (plunger 708). In addition, the fourth walls 450 regulate displacement of the plunger 708 in the direction of arrow Y with respect to the support 414 and regulates displacement of the plunger 708 (a plunger flange 746) in the direction of arrow X2 with respect to the support 414 (outer cylinder 712). While the prefilled syringe 702 is attached to the syringe support 424, the cap 714 is not in contact with the support 414 nor with the package body 12.

JP5756793B2 discloses a prefilled syringe in which a prefilled syringe and a needle unit are separated from each other when not in use. Such a prefilled syringe is provided with an inner cylinder (barrel) filled with a drug in advance and a cap detachably provided at a leading end portion of the inner cylinder. Although the prefilled syringe is transported while being stored in a package body of a package unit, the cap may touch the package unit and may be loosened due to vibration during transportation.

According to this reference example, the cap 714 is not in contact with the package unit 411, which prevents the cap 714 from being loosened due to vibration during transportation. In addition, the first projections 444 of the support 414 are inserted into (engaged with) the first opening portions 728. This arrangement prevents the prefilled syringe 702 from being displaced with respect to the package unit 411 and the cap 714 from contacting the package the package unit 411 due to vibration during transportation.

Furthermore, since the two first opening portions 728 face each other, it is possible to effectively prevent displacement of the leading end of the prefilled syringe 702 with respect to the package unit 411. Still further, since the first opening portions 728 are through holes for a visual check of a drug, the arrangement of the outer cylinder 712 is simplified.

In this reference example, the second projections 448 of the support 414 are inserted into the third opening portions 732 placed on a base end of the outer cylinder 712. Accordingly, the base end of the prefilled syringe 702 is prevented from being displaced with respect to the package unit 411. Furthermore, since the two third opening portions 732 face each other, it is possible to effectively prevent displacement of the base end of the prefilled syringe 702 with respect to the package unit 411. Still further, since the third opening portions 732 are through holes for assembling the plunger 708 inside the inner cylinder 710, the arrangement of the outer cylinder 712 is simplified.

According to this reference example, a screwing action between the threaded portion 718 (see Fig. 2) of the connection 720 provided at a leading end portion of the inner cylinder 710 and the threaded portion 716 (see Fig. 2) of the cap 714 enables easy operation of attaching and detaching the cap 714 to and from the connection 720. Even in this case, it is possible to prevent the cap 714 from touching the package unit 411 and being loosened due to vibration during transportation.

In this reference example, the support 414 includes the base 420 that holds positions of the first projections 444 inside the package body 12. This arrangement prevents the cap 714 from touching the package body 12 due to vibration during transportation.

In addition, the needle unit case 762 is supported by the needle unit case support 422. This arrangement prevents displacement of the needle unit case 762 with respect to the package unit 411. Furthermore, the needle unit case support 422 is provided on the base 420. This arrangement effectively prevents displacement of the needle unit case 762 with respect to the package unit 411.

Still further, the projecting portions 426 of the needle unit case support 422 are inserted into the recesses 792 of the needle unit case 762. Accordingly, projections 794 of the needle unit case 762 and the projecting portions 426 of the needle unit case support 422 are brought into contact with each other. This arrangement prevents rotation of the needle unit case 762 about the axis with respect to the support unit 414. Accordingly, it is possible to hold the information visible and displayed on the outer surface of the sealing film 764 in a fixed orientation. Therefore, when opening the package body 12, it is easy to visually check the information associated with the needle unit 760.

### [Summary of Reference Example]

The syringe package 10G according to the reference example is provided with the prefilled syringe system 700 that includes the prefilled syringe 702 and the needle unit 760 connectably separated from the prefilled syringe 702 when not in use. The syringe package 10G is provided with the package unit 411 including the package body 12 which houses the prefilled syringe system 700 and the support 414 which is disposed inside the package body 12 and supports the prefilled syringe system 700.

The prefilled syringe 702 includes the inner cylinder 710 filled with a drug, the outer cylinder 712 provided outside the inner cylinder 710, and the sealing cap (cap 714) detachably attached to the leading end portion of the inner cylinder 710. The recesses (first opening portions 728 and third opening portions 732) are formed on an outer periphery of the outer cylinder 712. The support 414 is provided with the projections (first projections 444 and second projections 448) that engage with the recesses. Engagement between the projections and the recesses prevents displacement of the prefilled syringe 702 with respect to the package unit 411. The cap 714 is spaced from the package unit 411.

According to such an arrangement, the cap 714 is not in contact with the package unit 411, which prevents the cap 714 from being loosened during transportation. In addition, the projections of the support 414 are engaged with the recesses of the outer cylinder 712. This arrangement prevents the prefilled syringe 702 from being displaced with respect to the package unit 411 and the cap 714 from contacting the package unit 411 due to vibration during transportation.

In the syringe package 10G, the recesses include the leading end recesses (the first opening portions 728) placed on the leading end of the outer cylinder 712, and the projections include the leading end projections (the first projections 444) engaged with the leading end recesses.

Such a configuration effectively prevents displacement of the leading end portion (cap 714) of the prefilled syringe 702 with respect to the package unit 411.

In the syringe package 10G, a plurality of leading end recesses (first opening portions 728) is provided in the outer cylinder 712 while facing each other, and a plurality of leading end projections (first projections 444) is provided to be engaged with the leading end recesses (first opening portions 728).

Such a configuration effectively prevents displacement of the leading end of the prefilled syringe 702 with respect to the package unit 411.

In the syringe package 10G, the leading end recesses (first opening portions 728) are through holes for a visual check of the drug inside the inner cylinder 710 from the outside of the outer cylinder 712.

According to such an arrangement, since the through holes for a visual check of the drug are used, the arrangement of the outer cylinder 712 is simplified.

In the syringe package 10G, the recesses include the base end recesses (the third opening portions 732) placed on the base end of the outer cylinder 712, and the projections include the base end projections (second projections 448) engaged with the base end recesses.

Such an arrangement prevents displacement of the base end of the prefilled syringe 702 with respect to the package unit 411.

In the syringe package 10G, a plurality of base end recesses (third opening portions 732) is provided in the outer cylinder 712 while facing each other, and a plurality of base end projections (second projections 448) is provided to be engaged with the base end recesses (third opening portions 732).

Such an arrangement effectively prevents displacement of the base end of the prefilled syringe 702 with respect to the package unit 411.

In the syringe package 10G, the base end recesses (third opening portions 732) are through holes for assembling of the plunger 708 inside the inner cylinder 710.

According to such an arrangement, since the through holes for assembling of the plunger are used, the arrangement of the outer cylinder 712 is simplified.

In the syringe package 10G, the leading end portion of the inner cylinder 710 is provided with the connection 720 including the first threaded portion (threaded portion 718), and the cap 714 includes the second threaded portion (threaded portion 716) which is screwed into the first threaded portion.

According to such an arrangement, a screwing action between the first threaded portion and the second threaded portion enables easy operation of attaching and detaching the cap 714 to and from the connection 720. Even in this case, it is possible to prevent the cap 714 from touching the package unit 411 and being loosened due to vibration during transportation.

In the syringe package 10G, the support 414 includes the base 420 that holds positions of the projections (the first projections 444 and the second projections 448) inside the package body 12.

Such an arrangement prevents the cap 714 from touching the package body 12 due to vibration during transportation.

In the syringe package 10G, the prefilled syringe system 700 is provided with the needle package 704 including the needle unit 760 that enables puncture of a living body and the needle unit case 762 that houses the needle unit 760. The support 414 includes the needle unit case support 422 that supports the needle unit case 762.

Such an arrangement prevents displacement of the needle package 704 with respect to the package unit 411.

In the syringe package 10G, the needle unit case support 422 is provided on the base 420.

Such an arrangement effectively prevents displacement of the needle package 704 with respect to the package unit 411.

In the syringe package 10G, the needle unit case 762 includes the case body 763 with the needle unit 760 provided therein and the sealing film 764 that seals the case body 763. The outer surface of the sealing film 764 displays the information visible and associated with the needle unit 760. An outer surface of the needle unit case 762 is provided with first locking units (the projections 794). The needle unit case support 422 includes the second locking units (the projecting portions 426) that are locked with the first locking unit to regulate displacement of the needle unit case 762 about the axis with respect to the support 414.

Such an arrangement regulates rotation of the needle unit case 762 along the axis with respect to the package unit 411, which makes it possible to hold the information visible and displayed on the outer surface of the sealing film 764 in a fixed orientation. Therefore, when opening the package body 12, it is easy to visually check the information associated with the needle unit 760.

It is a matter of course that the package according to the present invention is not limited to the embodiments described above and may employ various arrangements without departing from the gist of the present invention.

### Reference Signs List

10A to 10G SYRINGE PACKAGE
12, 150, 220 PACKAGE BODY
14, 90, 152, 222, 370, 414 SUPPORT
36, 106, 193, 246, 436 BASE END SUPPORT
700 PREFILLED SYRINGE SYSTEM
702 PREFILLED SYRINGE
704 NEEDLE PACKAGE
705 SYRINGE BODY
706 GASKET
708 PLUNGER
746 PLUNGER FLANGE
760 NEEDLE UNIT
762 NEEDLE UNIT CASE

## Claims

1. A syringe package (10A to 10F) provided with a prefilled syringe system (700) that includes a prefilled syringe (702) including a syringe body (705) which is filled with a drug in advance the syringe package (10A to 10F) comprising:
a package body (12, 150, 220) that houses the prefilled syringe (702); and
a support (14, 90, 152, 222, 370) disposed inside the package body (12, 150, 220) and configured to support the prefilled syringe (702),
wherein the support (14, 90, 152, 222, 370) includes a displacement regulation unit (36, 106, 193, 266, 375) configured to regulate relative displacement of the syringe body (705) and the plunger (708) in a direction in which the gasket (706) and the plunger (708) approach each other and that
within the syringe body (705) the gasket (706) and the plunger (708) are not connected to each other when the syringe package (10A to 10F) is not in use,
the gasket (706) and the plunger (708) are spaced from each other, and
the plunger (708) has a base end portion provided with a plunger flange (746), and
the displacement regulation unit (36, 106, 193, 266, 375) is disposed between the syringe body (705) and the plunger flange (746).

2. The syringe package (10A to 10F) according to claim 1, wherein the displacement regulation unit (36, 106, 193, 266, 375) is provided with a groove (66, 144, 184, 200, 262, 374e, 376c) into which at least a part of the plunger (708) is inserted.

3. The syringe package (10A to 10F) according to any one of claims 1 to 2,
wherein the support (14, 90, 152, 222, 370) includes a base (20, 92, 162, 228, 374a) configured to hold a position of the displacement regulation unit (36, 106, 193, 266, 375) inside the package body (12, 150, 220).

4. The syringe package (10A, 10C, 10D) according to claim 3,
wherein the support (14, 90, 222) includes a positional shift regulation unit (34, 104, 252) that contacts the syringe body (705) and regulates displacement of the syringe body (705) in a direction intersecting with an axis of the syringe body (705).

5. The syringe package (10A, 10C, 10D) according to claim 4,
wherein the positional shift regulation unit (34, 104, 252) locks the syringe body (705).

6. The syringe package (10A, 10C, 10D) according to claim 4 or 5,
wherein the positional shift regulation unit (34, 104, 252) is provided on the base (20, 92, 228).

7. The syringe package (10A to 10F) according to any one of claims 3 to 6,
wherein the support (14, 90, 152, 222, 370) includes a leading end regulation unit (32, 102, 164, 252, 374b) that contacts the syringe body (705) and regulates displacement of the syringe body (705) toward a leading end of the syringe package (10A to 10F).

8. The syringe package (10A to 10F) according to claim 7,
wherein the leading end regulation unit (32, 102, 164, 252, 374b) is provided on the base (20, 92, 162, 228, 374a).

9. The syringe package (10A to 10F) according to any one of claims 3 to 8,
wherein the prefilled syringe system (700) comprises:
a needle package (704) including a needle unit (760) that enables puncture of a living body and a needle unit case (762) that houses the needle unit (760); and
a prefilled syringe (702) including a connection (720) to which the needle unit (760) is connected, the syringe body (705), and the plunger (708),
wherein the support (14, 90, 152, 222, 370) includes a needle unit case support (22, 94, 210, 234, 376b) that supports the needle unit case (762).

10. The syringe package (10A to 10F) according to claim 9,
wherein the needle unit (760) and the prefilled syringe (702) are separated from each other when the syringe package (10A to 10F) is not in use.

11. The syringe package (10A, 10C, 10D) according to claim 9 or 10,
wherein the needle unit case support (22, 94, 234) is provided on the base (20, 92, 228).

## Patentansprüche

1. Spritzenverpackung (10A bis 10F), die mit einem vorgefüllten Spritzensystem (700) versehen ist, das eine vorgefüllte Spritze (702) umfasst, die einen Spritzenkörper (705) aufweist, der im Voraus mit einem Arzneimittel gefüllt ist, wobei die Spritzenverpackung (10A bis 10F) umfasst:
einen Verpackungskörper (12, 150, 220), der die vorgefüllte Spritze (702) aufnimmt; und
eine Halterung (14, 90, 152, 222, 370), die innerhalb des Verpackungskörpers (12, 150, 220) angeordnet und so konfiguriert ist, dass sie die vorgefüllte Spritze (702) hält,
wobei die Halterung (14, 90, 152, 222, 370) eine Verschiebungsregulierungseinheit (36, 106, 193, 266, 375) umfasst, die so konfiguriert ist, dass sie die relative Verschiebung des Spritzenkörpers (705) und des Kolbens (708) in einer Richtung reguliert, in der sich die Dichtung (706) und der Kolben (708) einander nähern,
und dass innerhalb des Spritzenkörpers (705) die Dichtung (706) und der Kolben (708) nicht miteinander verbunden sind, wenn die Spritzenverpackung (10A bis 10F) nicht in Gebrauch ist,
die Dichtung (706) und der Kolben (708) voneinander beabstandet sind, und
der Kolben (708) einen Basisendabschnitt aufweist, der mit einem Kolbenflansch (746) versehen ist, und
die Verschiebungsregulierungseinheit (36, 106, 193, 266, 375) zwischen dem Spritzenkörper (705) und dem Kolbenflansch (746) angeordnet ist.

2. Spritzenverpackung (10A bis 10F) nach Anspruch 1,
wobei die Verschiebungsregulierungseinheit (36, 106, 193, 266, 375) mit einer Nut (66, 144, 184, 200, 262, 374e, 376c) versehen ist, in die mindestens ein Teil des Kolbens (708) eingesetzt ist.

3. Spritzenverpackung (10A bis 10F) nach einem der Ansprüche 1 bis 2,
wobei die Halterung (14, 90, 152, 222, 370) eine Basis (20, 92, 162, 228, 374a) umfasst, die so konfiguriert ist, dass sie eine Position der Verschiebungsregulierungseinheit (36, 106, 193, 266, 375) innerhalb des Verpackungskörpers (12, 150, 220) hält.

4. Spritzenverpackung (10A, 10C, 10D) nach Anspruch 3,
wobei die Halterung (14, 90, 222) eine Positionsverschiebungsregulierungseinheit (34, 104, 252) umfasst, die mit dem Spritzenkörper (705) in Kontakt tritt und die Verschiebung des Spritzenkörpers (705) in einer Richtung reguliert, die sich mit einer Achse des Spritzenkörpers (705) schneidet.

5. Spritzenverpackung (10A, 10C, 10D) nach Anspruch 4,
wobei die Positionsverschiebungsregulierungseinheit (34, 104, 252) den Spritzenkörper (705) verriegelt.

6. Spritzenverpackung (10A, 10C, 10D) nach Anspruch 4 oder 5,
wobei die Positionsverschiebungsregulierungseinheit (34, 104, 252) an der Basis (20, 92, 228) vorgesehen ist.

7. Spritzenverpackung (10A bis 10F) nach einem der Ansprüche 3 bis 6,
wobei die Halterung (14, 90, 14, 90, 222) eine Regulierungseinheit (32, 102, 164, 252, 374b) für das vordere Ende aufweist, die mit dem Spritzenkörper (705) in Kontakt tritt und die Verschiebung des Spritzenkörpers (705) in Richtung eines vorderen Endes der Spritzenverpackung (10A bis 10F) reguliert.

8. Spritzenverpackung (10A bis 10F) nach Anspruch 7,
wobei die Regulierungseinheit (32, 102, 164, 252, 374b) für das vordere Ende an der Basis (20, 92, 162, 228, 374a) vorgesehen ist.

9. Spritzenverpackung (10A bis 10F) nach einem der Ansprüche 3 bis 8,
wobei das vorgefüllte Spritzensystem (700) umfasst:
eine Nadelverpackung (704), die eine Nadeleinheit (760), welche die Punktion eines lebenden Körpers ermöglicht, und ein Nadeleinheitsgehäuse (762) umfasst, welches die Nadeleinheit (760) aufnimmt; und
eine vorgefüllte Spritze (702), die einen Anschluss (720), mit welchem die Nadeleinheit (760) verbunden ist, den Spritzenkörper (705) und den Kolben (708) umfasst,
wobei die Halterung (14, 90, 152, 222, 370) eine Nadeleinheitsgehäusehalterung (22, 94, 210, 234, 376b) umfasst, die das Nadeleinheitsgehäuse (762) hält.

10. Spritzenverpackung (10A bis 10F) nach Anspruch 9,
wobei die Nadeleinheit (760) und die vorgefüllte Spritze (702) voneinander getrennt sind, wenn die Spritzenverpackung (10A bis 10F) nicht in Gebrauch ist.

11. Spritzenverpackung (10A, 10C, 10D) nach Anspruch 9 oder 10,
wobei die Nadeleinheitsgehäusehalterung (22, 94, 234) an der Basis (20, 92, 228) vorgesehen ist.

## Revendications

1. Boîtier de seringue (10A à 10F) pourvu d'un système de seringue préremplie (700) qui comporte une seringue préremplie (702) comportant un corps de seringue (705) qui est remplie par avance d'un médicament le boîtier de seringue (10A à 10F) comprenant :
un corps de boîtier (12, 150, 220) qui loge la seringue préremplie (702) ; et
un support (14, 90, 152, 222, 370) disposé à l'intérieur du corps de boîtier (12, 150, 220) et configuré pour supporter la seringue préremplie (702),
dans lequel le support (14, 90, 152, 222, 370) comporte une unité de régulation de déplacement (36, 106, 193, 266, 375) configurée pour réguler le déplacement relatif du corps de seringue (705) et du piston (708) dans une direction dans laquelle le joint d'étanchéité (706) et le piston (708) s'approchent l'un de l'autre et que dans le corps de seringue (705) le joint d'étanchéité (706) et le piston (708) ne sont pas reliés l'un à l'autre lorsque le boîtier de seringue (10A à 10F) n'est pas en cours d'utilisation,
le joint d'étanchéité (706) et le piston (708) sont espacés l'un de l'autre, et
le piston (708) présente une partie d'extrémité de base pourvue d'une bride de piston (746), et
l'unité de régulation de déplacement (36, 106, 193, 266, 375) est disposée entre le corps de seringue (705) et la bride de piston (746).

2. Boîtier de seringue (10A à 10F) selon la revendication 1,
dans lequel l'unité de régulation de déplacement (36, 106, 193, 266, 375) est pourvue d'une rainure (66, 144, 184, 200, 262, 374e, 376c) à l'intérieur de laquelle au moins une partie du piston (708) est insérée.

3. Boîtier de seringue (10A à 10F) selon l'une quelconque des revendications 1 à 2,
dans lequel le support (14, 90, 152, 222, 370) comporte une base (20, 92, 162, 228, 374a) configurée pour maintenir une position de l'unité de régulation de déplacement (36, 106, 193, 266, 375) à l'intérieur du corps de boîtier (12, 150, 220).

4. Boîtier de seringue (10A, 10C, 10D) selon la revendication 3,
dans lequel le support (14, 90, 222) comporte une unité de régulation de décalage de position (34, 104, 252) qui est en contact avec le corps de seringue (705) et régule le déplacement du corps de seringue (705) dans une direction coupant un axe du corps de seringue (705).

5. Boîtier de seringue (10A, 10C, 10D) selon la revendication 4,
dans lequel l'unité de régulation de décalage de position (34, 104, 252) verrouille le corps de seringue (705).

6. Boîtier de seringue (10A, 10C, 10D) selon la revendication 4 ou 5,
dans lequel l'unité de régulation de décalage de position (34, 104, 252) est prévue sur la base (20, 92, 228).

7. Boîtier de seringue (10A à 10F) selon l'une quelconque des revendications 3 à 6,
dans lequel le support (14, 90, 152, 222, 370) comporte une unité de régulation d'extrémité avant (32, 102, 164, 252, 374b) qui est en contact avec le corps de seringue (705) et régule le déplacement du corps de seringue (705) en direction d'une extrémité avant du boîtier de seringue (10A à 10F).

8. Boîtier de seringue (10A à 10F) selon la revendication 7,
dans lequel l'unité de régulation d'extrémité avant (32, 102, 164, 252, 374b) est prévue sur la base (20, 92, 162, 228, 374a).

9. Boîtier de seringue (10A à 10F) selon l'une quelconque des revendications 3 à 8,
dans lequel le système de seringue préremplie (700) comprend :
un boîtier d'aiguille (704) comportant une unité d'aiguille (760) qui permet de piquer un corps vivant et
un étui d'unité d'aiguille (762) qui loge l'unité d'aiguille (760) ; et
une seringue préremplie (702) comportant une liaison (720) à laquelle l'unité d'aiguille (760) est reliée, le corps de seringue (705), et le piston (708),
dans lequel le support (14, 90, 152, 222, 370) comporte un support d'étui d'unité d'aiguille (22, 94, 210, 234, 376b) qui supporte l'étui d'unité d'aiguille (762).

10. Boîtier de seringue (10A à 10F) selon la revendication 9,
dans lequel l'unité d'aiguille (760) et la seringue préremplie (702) sont séparées l'une de l'autre lorsque le boîtier de seringue (10A à 10F) n'est pas en cours d'utilisation.

11. Boîtier de seringue (10A, 10C, 10D) selon la revendication 9 ou 10,
dans lequel le support d'étui d'unité d'aiguille (22, 94, 234) est prévu sur la base (20, 92, 228).
